(19) Europäisches Patentamt
European Patent Office
Office européen des brevets

(11) Publication number: 0 467 850 B1

(12) # EUROPEAN PATENT SPECIFICATION

(45) Date of publication of patent specification: **06.09.95**

(51) Int. Cl.[6]: **C07C 69/54**, C07C 57/075, C07C 15/46, C07C 67/62, C07C 7/20

(21) Application number: **91810561.0**

(22) Date of filing: **11.07.91**

(54) **Stabilized monomer compositions.**

(30) Priority: **20.07.90 US 556066**

(43) Date of publication of application:
**22.01.92 Bulletin 92/04**

(45) Publication of the grant of the patent:
**06.09.95 Bulletin 95/36**

(84) Designated Contracting States:
**DE FR GB IT**

(56) References cited:
**EP-A- 0 334 500**
**US-A- 4 016 198**
**US-A- 4 668 721**
**US-A- 4 691 015**
**US-A- 4 912 247**

(73) Proprietor: **CIBA-GEIGY AG**
**Klybeckstrasse 141**
**CH-4002 Basel (CH)**

(72) Inventor: **Gatechair, Leslie R.**
**Rural Route 2,**
**Box 60**
**Katonah,**
**New York 10536 (US)**
Inventor: **Seltzer, Raymond**
**11 Angus Lane**
**New City, N.Y. 10956 (US)**
Inventor: **Hyun, James Lee**
**6 Kevin Drive**
**Danbury,**
**Conn. 06811 (US)**

**EP 0 467 850 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The instant invention pertains to stabilized monomer compositions, in particular to compositions containing ethylenically unsaturated monomers, stabilized against premature polymerization.

The ethylenically unsaturated compounds which can be polymerized by free radical initiation are commonly called monomers. They constitute a major class of industrial chemicals. Because of the presence of the polymerizable double bond, the widespread sources of initiating radicals from peroxides, light and/or thermal generation, such monomers are prone to undesirable and premature polymerization at various stages during their manufacture, purification, storage, shipping, blending and use. Protection of such monomers from such premature polymerization is needed up to the point where polymerization is actually desired. If premature polymerization does occur, the monomer may suffer contamination by polymer, troublesome increase in viscosity, gelation and/or loss of reactivity. Fouling of distillation equipment including heat exchanger surfaces, storage vessels, transfer lines, pumps, shipping containers and application equipment can occur with ensuing costs of cleaning, downtime, loss of material and unnecessary labor costs. A particularly difficult situation is the preparation of polyol acrylates from polyols and acrylic acid since prolonged heating periods are required to complete the esterification. Premature polymerization can also constitute a safety hazard since uncontrolled exothermic polymerization can cause ruptured vessels, atmospheric contamination, and in extreme cases, explosions and fires. Deterioration of monomers in shipping and storage may also make necessary the use of costly refrigerated shipping and storage facilities.

A further problem is that of undesired polymerization of adventitious monomers, that is, radically-polymerizable unsaturated monomers which occur in commercial products such as hydrocarbon fuels and refinery streams. In these cases, polymerization accompanied by the incorporation of oxygen moieties leads to gum and sludge deposits which can foul carburetors, engines, fuel tanks or fuel lines. In refineries, the adventitious monomers in hydrocarbon streams such as cracking products can foul pipelines, valves, pumps, heat exchangers, stills and storage vessels.

Another problem in regard to undesired polymerization of free radical polymerizable monomers is the case of polymerizations which are intentional, but which must be prevented from going too far. For example, the quality of poly(vinyl chloride) suspension polymer and of synthetic rubber made from olefins and dienes is superior (i.e. better molecular weight distribution, stability, and processing properties) if the polymerization is stopped short of complete consumption of the monomers. It is also desirable to have available in a plant conducting vinyl polymerization reactions some rapid and efficient means for stopping a runaway polymerization if other means such as cooling should fail.

It is known that the addition of certain compounds to monomers can retard or even prevent their undesired polymerization, and that when polymerization of the monomer is desired, the inhibitor can be removed or overridden by a deliberately-added polymerization initiator. Various aromatic compounds have been used as such inhibitors in the prior art. Typical ones are hydroquinone, monomethyl ether of hydroquinone (MEHQ), tert-butylphenols, phenothiazine, phenylenediamines and benzoquinones. These are usually used at a level of 50 to 1000 ppm. These inhibitors are not totally effective, and even with such inhibitors present, it is often advisable to store such inhibited monomers in a cool place and for limited periods of time. Moreover, these aromatic inhibitors are a cause of serious discoloration problems in the monomers and in polymers deliberately prepared from such monomers. Typically these aromatic inhibitors produce quinoidal chromophoric groups with very high visible light absorbance. The use of stable nitroxyl radicals as inhibitors also leads to discoloration since such compounds are themselves highly colored, usually bright red.

In order to overcome these color problems, a diligent search was made to find alternative inhibitors which are both effective and not discoloring. This search led to the N,N-dialkylhydroxylamines and the N,N-diaralkylhydroxylamines. Some typical references are cited infra.

US-A-3,222,334 and US-A-3,878,181 disclose the use of N,N-dialkylhydroxylamines such as N,N-diethylhydroxylamine as short-stopping agents for emulsion polymerizations of butadiene/styrene rubber and chloroprene.

US-A-3,148,225 and US-A-3,697,470 disclose the use of N,N-dialkylhydroxylamines such as N,N-diethylhydroxylamine and N-alkyl-N-arylhydroxylamine such as N-ethyl-N-phenylhydroxylamine respectively as short-stopping agents and popcorn polymer inhibitors in processes for preparing synthetic rubber. The popcorn polymer formation is a serious problem encountered in recovering of monomers from such synthetic rubber operations.

US-A-4,782,105 teaches the use of long chain N,N-dialkylhydroxylamines as stabilizers to prevent the premature gelation of unsaturated elastomer compositions such as styrene/butadiene copolymers or

polybutadiene.

US-A-3,408,422 describes the use of N,N-dialkylhydroxylamines such as N,N-diethylhydroxylamine and N,N-diaralkylhydroxylamines such as N,N-dibenzylhydroxylamine as stabilizers for preventing the premature gelation of unsaturated polyesters.

US-A-4,798,889 teaches the use of N,N-dialkylhydroxylamines such as N,N-diethylhydroxylamine or N,N-dibenzylhydroxylamine as stabilizers to reduce the thermal polymerization of organosiloxanes substituted by ethylenically unsaturated moieties.

US-A-4,409,408 and US-A-4,434,307 disclose the use of N,N-dibenzylhydroxylamine in combination with an alkylated diphenol (catechol or hydroquinone) as inhibitors to prevent the polymerization of styrene.

The use of stable nitroxyl radicals including those derived from hindered amine moieties has also been disclosed. Typical references are cited below.

SU-A-1,139,722 describes the inhibition of styrene and comonomers such as butadiene using 1-oxyl derivatives of hindered amine compounds such as N,N'-bis(1-oxyl-2,2,6,6-tetramethylpiperidin-4-yl)-adipamide The elimination of popcorn polymer and of the clogging of equipment is touted as the result of using such 1-oxyl compounds.

JP-A-60-36501 describes the use of hindered amines and their 1-oxyl and 1-alkyl derivatives as vinyl polymerization inhibitors to improve storage stability of monomers such as acrylate and methacrylate esters.

EP-A-178,168 and GB-A-1,127,127 describe the use of 1-oxyl substituted hindered amine compounds as stabilizers for inhibiting the polymerization of $\alpha,\beta$-ethylenically unsaturated monocarboxylic acids, such as acrylic acid, during its recovery by distillation.

US-A-4,670,131 teaches the use of 1-oxyl substituted hindered amine compounds as stabilizers for preventing the fouling of equipment for processing organic feed streams containing olefins by inhibiting the polymerization of said olefins.

In a theoretical study of the inhibiting effects of selected hindered amine compounds, Y. Miura et al., Makromol. Chem. 160, 243 (1972) disclose that 1-oxyl-2,2,6,6-tetramethylpiperidin-4-one is highly effective in retarding the onset of the polymerization of styrene and methyl methacrylate. By contrast, the corresponding 1-benzyloxy-2,2,6,6-tetramethylpiperidin-4-one is stated to have no effect in delaying the polymerization of styrene and no retarding effect on said polymerization once begun.

US-A-4,668,721 and US-A-4,691,015 disclose the use of 1-hydroxy substituted hindered amine compounds as stabilizers for polyolefin compositions in combination with one or more other stabilizers such as phenolic antioxidants, ultraviolet light absorbers and the like.

EP-A-334,500 (Derwent 89-279844/39) describes polymerization inhibition compositions comprising (a) a phenothiazine and (b) a substituted phenylenediamine for inhibiting the polymerization of styrene.

US-A-4,016,198 describes the use of a polyalkylamine and an arylenediamine for stabilization of unsaturated carboxylic esters.

US-A-4,912,247 describes a method for inhibiting the polymerization of acrylates by adding to said acrylate an amine obtained by the condensation reaction from a phenol, a aldehyde and ethylenediamine (Mannich product), and a phenylenediamine and/or phenotiazine derivative.

None of these references describes or suggests that a substituted hindered amine plus phenothiazine or other related heterocyclic moiety is or could possibly be such effective inhibitors to prevent the premature polymerization of monomers in either the liquid or vapor phase.

It is the broad object of the invention to provide monomer compositions inhibited against undesired and premature polymerization by means of small, but effective amounts of selected additives which do not impart undesired color to the monomer compositions.

It is a further object of the invention to provide inhibited monomer compositions which have substantially improved stability relative to compositions inhibited by methods known in the prior art.

It is a further object of the invention to provide a means for short-stopping or retarding polymerization of monomers once polymerization is started.

It is a further object of the invention to provide effective inhibitors for monomers known to be difficult to inhibit such as acrylic acid.

It is still a further object of the invention to provide highly effective combinations of inhibitors for said monomers.

The instant invention pertains to a monomer composition stabilized against premature polymerization, which comprises

(a) an ethylenically unsaturated monomer or mixture of monomers, polymerizable by free radical initiation, and

(b) an effective amount, sufficient to inhibit premature polymerization of component (a), of a combination of

(i) a heterocyclic compound selected from any of formulas A to D

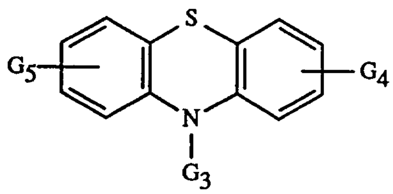

(A)

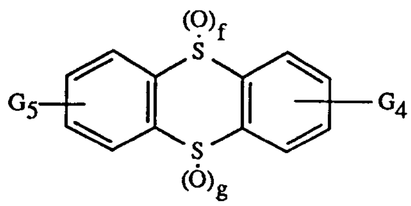

(B)

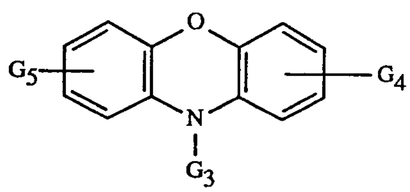

(C)

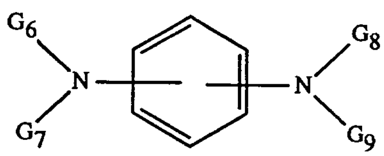

(D)

where

$G_3$ is hydrogen, alkyl of 1 to 4 carbon atoms or alkenyl of 3 to 4 carbon atoms,

$G_4$ and $G_5$ are independently hydrogen or alkyl of 1 to 8 carbon atoms,

$G_6$ is aryl of 6 to 10 carbon atoms or aralkyl of 7 to 15 carbon atoms, and

$G_7$, $G_8$ and $G_9$ are independently hydrogen, alkyl of 1 to 18 carbon atoms, cycloalkyl of 5 to 12 carbon atoms, aralkyl of 7 to 15 carbon atoms or alkaryl of 7 to 15 carbon atoms;

f and g are independently 0, 1 or 2, and

(ii) a compound or mixture of compounds of any of formulas I to XIX

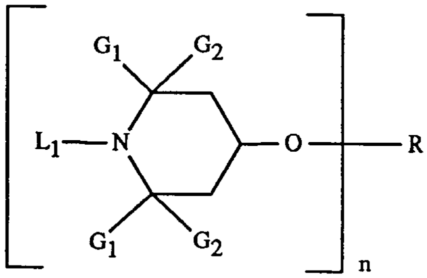

(I)

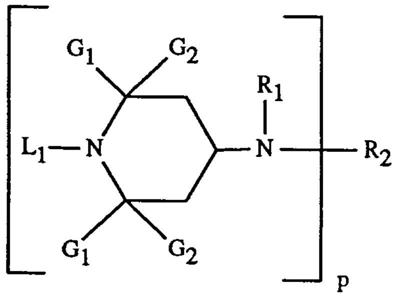

(II)

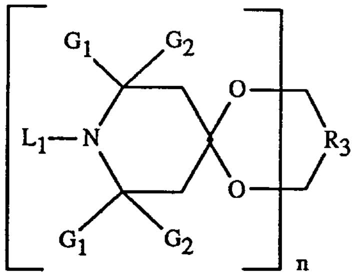

(III)

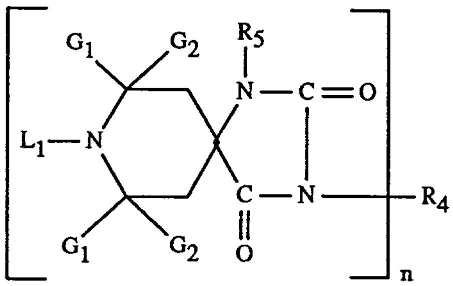

(IV)

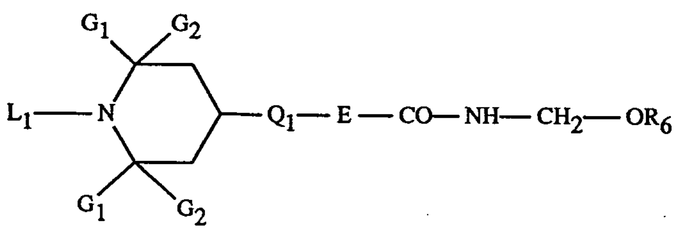

(V)

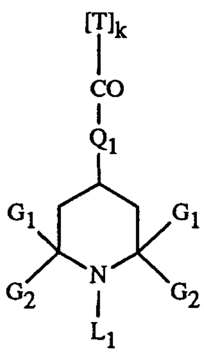

(VI)

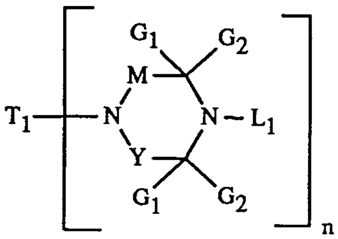

(VII)

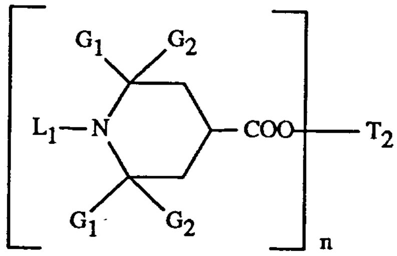

(VIII)

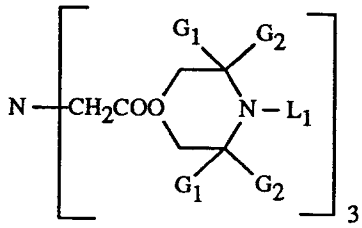

(IX)

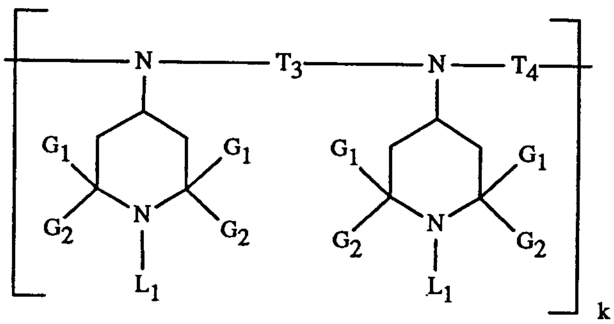

(X)

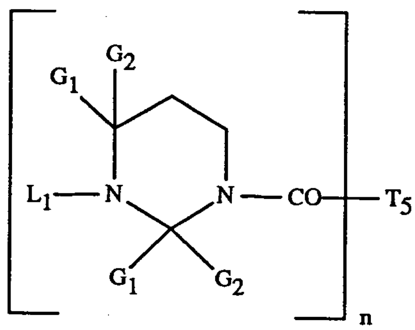

(XI)

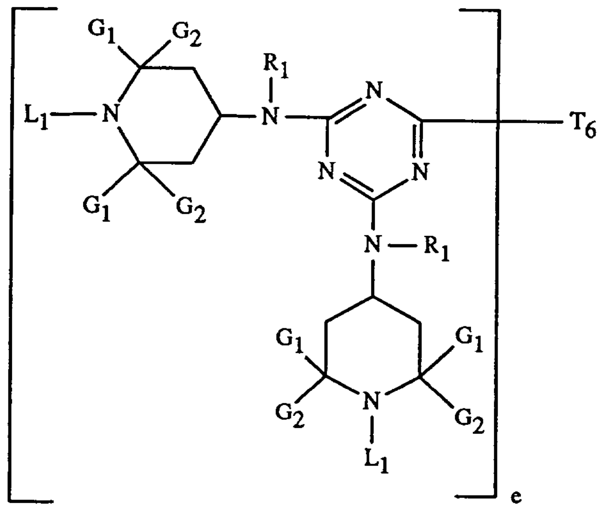

(XII)

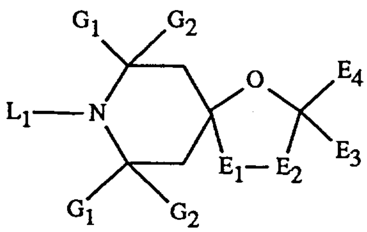

(XIII)

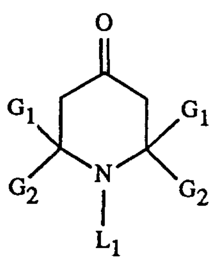

(XIV)

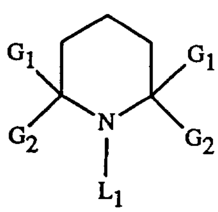

(XV)

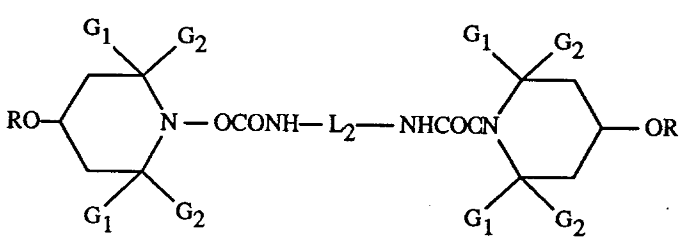

XVI

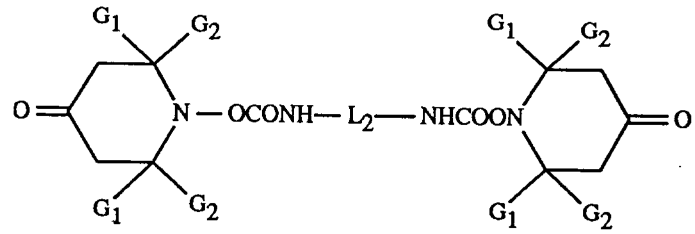

XVII

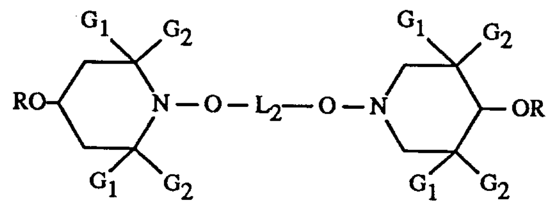

XVIII

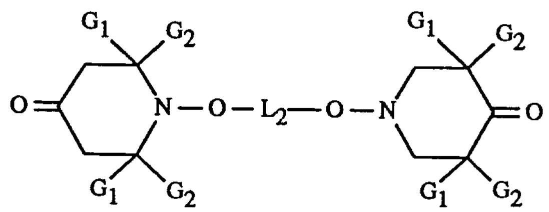

XIX

wherein

$G_1$ and $G_2$ are independently alkyl of 1 to 4 carbon atoms;

$L_1$ is hydrogen, hydroxyl, alkyl of 1 to 18 carbon atoms, said alkyl substituted by hydroxyl, cyanoethyl, glycidyl, aralkyl of 7 to 15 carbon atoms or a monovalent acyl radical of an aliphatic, cycloaliphatic, araliphatic or aromatic acid, $-OCONHL_3$ or -OL4, where

$L_3$ is hydrogen, alkyl of 2 to 18 carbon atoms, allyl, cyclohexyl, aryl of 6 to 10 carbon atoms, said aryl substituted by one or two alkyl groups of 1 to 4 carbon atoms or is benzyl,

$L_4$ is alkyl of 1 to 18 carbon atoms, cycloalkyl of 5 to 12 carbon atoms, alkenyl of 2 to 18 carbon atoms, cycloalkenyl of 5 to 12 carbon atoms, aralkyl of 7 to 15 carbon atoms, a radical of a saturated or unsaturated bicyclic or tricyclic hydrocarbon of 7 to 12 carbon atoms or aryl of 6 to 10 carbon atoms or said aryl substituted by alkyl of 1 to 4 carbon atoms, or $L_4$ is $-CH_2CH_2COOL_5$ where $L_5$ is alkyl of 1 to 18 carbon atoms,

n is 1 or 2,

when n is 1,

R is hydrogen, $C_1$-$C_{18}$-alkyl optionally interrupted by one or more oxygen atoms, cyanoethyl, benzyl, glycidyl, a monovalent acyl radical of a aliphatic, cycloaliphatic, araliphatic or aromatic acid, or of carbamic acid or of a phosphorus-containing acid, or a monovalent silyl radical; or

when n is 2,

R is $C_1$-$C_{12}$-alkylene, $C_4$-$C_{12}$-alkenylene, xylylene, a divalent acyl radical of an aliphatic, cycloaliphatic, araliphatic or aromatic dicarboxylic acid, or of a dicarbamic acid or of a phosphorus-containing acid, or a bivalent silyl radical;

p is 1, 2 or 3,

$R_1$ is $C_1$-$C_{12}$-alkyl, $C_5$-$C_7$-cycloalkyl, $C_7$-$C_8$-aralkyl, $C_2$-$C_{18}$-alkanoyl, $C_3$-$C_5$-alkenoyl or benzoyl;

when p is 1,

$R_2$ is $C_1$-$C_{18}$-alkyl, $C_5$-$C_7$-cycloalkyl, $C_2$-$C_8$-alkenyl unsubstituted or substituted by a cyano, carbonyl or carbamide group, or is glycidyl, a group of the formula $-CH_2CH(OH)$-Z or of the formula -CONH-Z wherein Z is hydrogen, methyl or phenyl; or when p is 2,

$R_2$ is $C_2$-$C_{12}$-alkylene, $C_6$-$C_{12}$-arylene, xylylene, a $-CH_2CH(OH)CH_2$-O-X-O-$CH_2CH(OH)CH_2$- wherein X is $C_2$-$C_{10}$-alkylene, $C_6$-$C_{15}$-arylene or $C_6$-$C_{12}$-cycloalkylene; or, provided that $R_1$ is not alkanoyl, alkenoyl or benzoyl, $R_2$ can also be a divalent acyl radical of an aliphatic, cycloaliphatic or aromatic dicarboxylic acid or dicarbamic acid, or can be the group -CO-; or $R_1$ and $R_2$ together when p is 1 can be the cyclic acyl radical of an aliphatic or aromatic 1,2- or 1,3-dicarboxylic acid; or

$R_2$ is

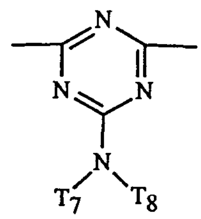

where $T_7$ and $T_8$ are independently hydrogen, alkyl of 1 to 18 carbon atoms, or $T_7$ and $T_8$ together are alkylene of 4 to 6 carbon atoms or 3-oxapentamethylene;
when p is 3,
$R_2$ is 2,4,6-triazinyl;
when n is 1,
$R_3$ is $C_2$-$C_8$-alkylene or hydroxyalkylene or $C_4$-$C_{22}$-acyloxyalkylene; or
when n is 2,
$R_3$ is $(-CH_2)_2C(CH_2-)_2$;
when n is 1,
$R_4$ is hydrogen, $C_1$-$C_{12}$-alkyl, $C_3$-$C_5$-alkenyl, $C_7$-$C_9$-aralkyl, $C_5$-$C_7$-cycloalkyl,
$C_2$-$C_4$-hydroxyalkyl, $C_2$-$C_6$-alkoxyalkyl, $C_6$-$C_{10}$-aryl, glycidyl, a group of formula $-(CH_2)_m$-COO-Q or of the formula $-(CH_2)_m$-O-CO-Q wherein m is 1 or 2 and Q is $C_1$-$C_4$-alkyl or phenyl; or
when n is 2,
$R_4$ is $C_2$-$C_{12}$-alkylene, $C_6$-$C_{12}$-arylene, a group
$-CH_2CH(OH)CH_2$-O-X-O-$CH_2CH(OH)CH_2$- wherein X is $C_2$-$C_{10}$-alkylene,
$C_6$-$C_{15}$-arylene or $C_6$-$C_{12}$-cycloalkylene, or a group
$-CH_2CH(OZ_1)CH_2-(OCH_2CH(OZ_1)CH_2)_2$- wherein $Z_1$ is hydrogen, $C_1$-$C_{18}$-alkyl, allyl, benzyl, $C_2$-$C_{12}$-alkanoyl or benzoyl;
$R_5$ is hydrogen, $C_1$-$C_{12}$-alkyl, allyl, benzyl, glycidyl or $C_2$-$C_6$-alkoxyalkyl;
$Q_1$ is $-N(R_7)$- or -O-;
E is $C_1$-$C_3$-alkylene, the group $-CH_2CH(R_8)$-O- wherein $R_8$ is hydrogen, methyl or phenyl, the group $-(CH_2)_3$-NH- or a direct bond;
$R_7$ is $C_1$-$C_{18}$-alkyl, $C_5$-$C_7$-cycloalkyl, $C_7$-$C_{12}$-aralkyl, cyanoethyl, $C_6$-$C_{10}$-aryl, the group $-CH_2CH(R_8)$-OH; or a group of the formula

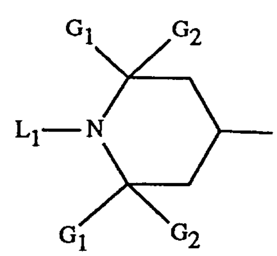

or a group of the formula

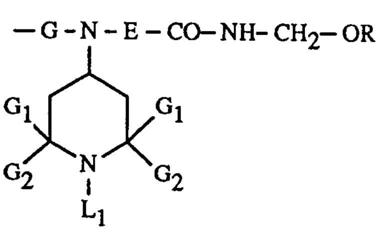

wherein G is $C_2$-$C_6$-alkylene or $C_6$-$C_{12}$-arylene; or
$R_7$ is a group -E-CO-NH-$CH_2$-$OR_6$;
$R_6$ is hydrogen or $C_1$-$C_{18}$-alkyl;
Formula VI denotes a recurring structural unit of a polymer where T is ethylene or 1,2-propylene, or is a repeating structural unit derived from an $\alpha$-olefin copolymer with an alkyl acrylate or methacrylate;
k is 2 to 100;
$T_1$ has the same meaning as $R_2$ when p is 1 or 2;
M and Y are independently methylene or carbonyl;
$T_2$ has the same meaning as $R_4$, and $T_2$ is octamethylene;
$T_3$ and $T_4$ are independently alkylene of 2 to 12 carbon atoms, of $T_4$ is

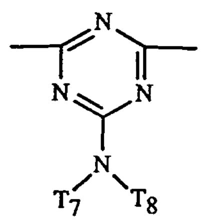

$T_6$ is

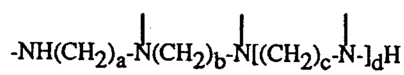

where a, b and c are independently 2 or 3, and d is 0 or 1;
e is 3 or 4;
$T_5$ is the same as R with the proviso that $T_5$ cannot be hydrogen when n is 1;
$E_1$ and $E_2$, being different, are each oxo or imino;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms; and
$E_4$ is hydrogen, alkyl of 1 to 30 carbon atoms, phenyl, naphthyl or phenylalkyl of 7 to 12 carbon atoms; or
$E_3$ and $E_4$ together are polymethylene of 4 to 17 carbon atoms, or said polymethylene substituted by up to four alkyl groups of 1 to 4 carbon atoms;
$L_2$ is a divalent radical from an aliphatic, cycloaliphatic or aromatic diisocyanate from which the two -NCO groups are removed, or is an alkanediyl of 1 to 18 carbon atoms or cyclohexanediyl.

The monomers of component (a) of this invention are any having at least one carbon-carbon double bond capable of undergoing free radical induced polymerization. Such monomers are well known in commerce and comprise a wide variety of structural types. Typical examples of such monomers are the olefinic hydrocarbons such as styrene, $\alpha$-methylstyrene and divinylbenzene; dienes such as butadiene and isoprene; halogenated monomers such as vinyl chloride, chloroprene, vinylidene chloride, vinylidene fluoride and vinyl fluoride; unsaturated acids such as acrylic acid, methacrylic acid and crotonic acid; unsaturated esters such as vinyl acetate, alkyl acrylates and alkyl methacrylates such as methyl methacrylate, ethyl acrylate, methyl acrylate, 2-hydroxyethyl acrylate and methacrylate, ethylene bismethacrylate, trimethylolpropane triacrylate, acrylated epoxy resin and polyethylene glycol diacrylate; unsaturated amides such as acrylamide, N,N-dimethylacrylamide, methylene-bisacrylamide and N-vinylpyrrolidone; unsaturated nitrile monomers such as acrylonitrile; and unsaturated ethers such as methyl vinyl ether; and miscellaneous monomers such as the vinyl pyridines, diethyl vinylphosphonate and sodium styrenesulfonate.

The instant invention also pertains to the use of mixtures of said monomers and to the use of resins such as acrylate-terminated polyurethanes and unsaturated polyesters. The common feature making all of these materials relevant to the present invention is the presence of a polymerizable double bond.

Also in the category of monomers are unsaturated oils such as drying oils like linseed oil, where polymerization also incorporates oxygen. There are also adventitious monomers formed in refining processes, for example polymerizable olefinic unsaturation in gasoline, jet fuel, solvents, crude oil and cracked hydrocarbon streams. The common feature of all of these substances is encompassed in the broad term

"monomers" and all are contemplated to be within the scope of instant component (a). Polymerization of such materials is often accompanied by autooxidation.

The acrylates, particularly acrylic acid itself, are unusually difficult to inhibit because of their inherent high polymerizability. The instant compounds are shown to be particularly effective in inhibiting acrylic acid from premature polymerization.

Preferably component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

Most preferably the monomer of component (a) is styrene, butadiene, vinyl chloride, acrylic acid, methacrylic acid, vinyl acetate, 2-hydroxyethyl acrylate, 2-hydroxyethyl methacrylate, trimethylolpropane triacrylate, polyethylene glycol diacrylate or methyl methacrylate.

Still more preferably the monomer is styrene, butadiene, acrylic acid or methacrylic acid.

The N-hydrocarbyloxy derivatives useful in the instant invention are denoted by the various structures of formulas I to XV, where I and XV are preferred. Another group of preferred derivatives are
1-[2-(methoxycarbonyl)ethoxy]-4-benzyloxy-2,2,6,6-tetramethyl piperidine;
1-methoxy-2,2,6,6-tetramethylpiperidin-4-yl benzoate;
bis[1-(2-(methoxycarbonyl)ethoxy)-2,2,6,6-tetramethylpiperidi n-4-yl] phthalate;
1-tert-butoxy-2,2,6,6-tetramethylpiperidin-4-yl benzoate;
4-hydroxy-1-octyloxy-2,2,6,6-tetramethylpiperidine;
1-[2-(methoxycarbonyl)ethoxy]-2,2,6,6-tetramethylpiperidine; 1-methylcyclohexyloxy-2,2, 6,6-tetramethylpiperidin-4-yl benzoate;
4-benzyloxy-1-ethoxy-2,2,6,6-tetramethylpiperidine;
1-carbamoyloxy-4-benzoyloxy-2,2,6,6-tetramethylpiperidine;
4-hydroxy-1,2,2,6,6-pentamethylpiperidine;
1-butylcarbamoyloxy-4-benzoyloxy-2,2,6,6-tetramethylpiperidine;
1-$\alpha$-methylbenzyloxy-2,2,6,6-tetramethylpiperidin-4-yl benzoate;
1,4-dimethoxy-2,2,6,6-tetramethylpiperidine;
bis[1-(2-(methoxycarbonyl)ethoxy)-2,2,6,6-tetramethylpiperidin-4-yloxy]-p-xylylene;
1-hydroxy-2,2,6,6-piperidin-4-yl benzoate;
4-hydroxy-1-methoxy-2,2,6,6-tetramethylpiperidine;
1-methoxy-2,2,6,6-tetrrmethylpiperidin-4-one;
1-hydroxy-2,2,6,6-tetramethylpiperidin-4-one;
4-hydroxyethoxy-2,2,6,6-tetramethylpiperidine;
bis(1-hydroxy-2,2,6,6-tetramethylpiperidin-4-yl) sebacate; and
bis(1-octyloxy-2,2,6,6-tetramethylpiperidin-4-yl) sebacate. A preferred component (i) is of formula (A) and is preferably phenothiazine. Most of these N-hydrocarbyloxy derivatives are known compounds. The instant N-hydrocarbyloxy derivatives can be easily prepared from the corresponding hindered amines which are known or which can be made by known procedures.

The instant N-hydrocarbyloxy derivatives are made by reacting a hydroxylamine with an alkyl halide or benzyl halide or reacting the hydroxylamine with a alkyl in presence of potassium butoxide.

Another method involves the preparation of the N-hydrocarbyloxy compounds directly from the hindered amine precursors using aqueous tert-butyl hydroperoxide, molybdenum trioxide in a appropriate hydrocarbon medium.

If any of the substituents in the above formulae are $C_1$-$C_{12}$-alkyl, they are for example methyl, ethyl, n-propyl, isopropyl, n-butyl, sec-butyl, tert-butyl, n-amyl, tert-amyl, n-hexyl, n-octyl, 2-ethylhexyl, tert-octyl, n-nonyl, n-decyl, n-undecyl or n-dodecyl. As $C_1$-$C_{18}$-alkyl, R can be the aforementioned groups, and in addition for example n-tridecyl, n-tetradecyl, n-hexadecyl or n-octadecyl.

$L_1$ is particularly hydrogen, hydroxyl, alkyl of 1 to 12 carbon atoms, $-OCONHL_3$ where $L_3$ is hydrogen, alkyl of 2 to 8 carbon atoms or phenyl, or $-OL_4$ where $L_4$ is particularly alkyl of 1 to 12 carbon atoms, cycloalkyl of 5 to 8 carbon atoms or is $-CH_2CH_2COOL_5$ where $L_5$ is particularly alkyl of 1 to 12 carbon atoms.

If R is a monovalent acyl radical of a carboxylic acid, it is for example a acyl radical of acetic acid, stearic acid, salicylic acid, methacrylic acid, acrylic acid, maleic acid, benzoic acid, 2-ethylhexanoic acid or 3,5-di-tert-butyl-4-hydroxyhydrocinnamic acid.

If R is a divalent acyl radical of a dicarboxylic acid, it is for example a acyl radical of adipic acid, succinic acid, suberic acid, sebacic acid, o-phthalic acid, butylmalonic acid, dibutylmalonic acid, dibenzylmalonic acid, 3,5-di-tert-butyl-4-hydroxybenzyl-butyl-malonic acid or bicycloheptene dicarboxylic acid.

If R is a divalent acyl radical of a dicarbamic acid, it is for example an acyl radical of hexamethylenedicarbamic acid or 2,4-toluylenedicarbamic acid.

R is also a acyl radical of a phosphorus-containing acid of the formula

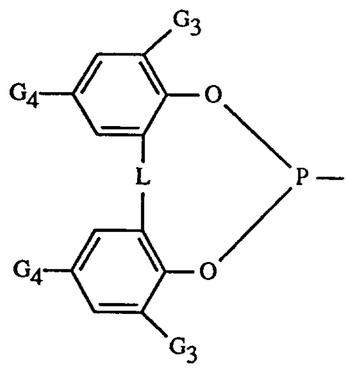

wherein L is a direct bond, methylene or alkylidene of 2 to 6 carbon atoms such as ethylidene, butylidene or amylidene. Preferably L is a direct bond, methylene or ethylidene.

$G_3$ and $G_4$ are independently alkyl of 1 to 4 carbonatoms, preferably methyl or tert-butyl. Most preferably $G_3$ and $G_4$ are each tert-butyl, or $G_3$ is tert-butyl and $G_4$ is methyl.

If any substituents are $C_5$-$C_7$-cycloalkyl, they are in particular cyclohexyl.

As $C_7$-$C_8$-aralkyl, $R_1$ is phenethyl and especially benzyl.

As $C_2$-$C_{18}$-alkanoyl, $R_1$ is for example propionyl, butyryl, octanoyl, lauroyl, hexadecanoyl, octadecanoyl, but especially acetyl; and as $C_3$-$C_5$-alkenoyl, $R_1$ is in particular acryloyl.

If $R_2$ is $C_2$-$C_8$-alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

When $R_1$ and $R_2$ are together a cyclic acyl radical, they are especially $-CO-(CH_2)_5-$.

If any substituents are $C_2$-$C_{12}$-alkylene, they are for example ethylene, propylene, 2,2,-dimethylpropylene, tetramethylene, hexamethylene, octamethylene, decamethylene or dodecamethylene.

If any substituents are $C_6$-$C_{15}$-arylene, they are for example o-, m- or p-phenylene, 1,4-naphthylene or 4,4'-diphenylene.

As $C_6$-$C_{12}$-cycloalkylene, X is especially cyclohexylene.

If $R_3$ is $C_2$-$C_8$-alkylene or hydroxyalkylene, it is for example ethylene, 1-methyl-ethylene propylene, 2-ethylpropylene or 2-ethyl-2-hydroxymethylpropylene.

As $C_4$-$C_{22}$acyloxyalkylene, $R_3$ is for example 2-ethyl-2-acetoxymethyl-propylene.

If any substituents are $C_2$-$C_6$-alkoxyalkyl, they are example methoxymethyl, ethoxymethyl, propoxymethyl, tert-butoxymethyl, ethoxyethyl, ethoxypropyl, n-butoxyethyl, tert-butoxyethyl, isopropoxyethyl or propoxypropyl.

If $R_4$ is $C_3$-$C_5$-alkenyl, it is for example 1-propenyl, allyl, methallyl, 2-butenyl or 2-pentenyl.

As $C_7$-$C_9$-aralkyl, $R_4$ is phenethyl or especially benzyl; and as $C_5$-$C_7$-cyclohexyl is especially cyclohexyl.

If $R_4$ is $C_2$-$C_4$-hydroxyalkyl, it is for example 2-hydroxyethyl, 2-hydroxypropyl, 3-hydroxypropyl, 2-hydroxybutyl or 4-hydroxybutyl.

As $C_6$-$C_{10}$-aryl, $R_4$ is in particular phenyl or $\alpha$- or $\beta$-naphthyl which is unsubstituted or substituted by halogen or $C_1$-$C_4$-alkyl.

If $R_4$ is $C_2$-$C_{12}$-alkylene, it is for example ethylene, propylene, 2,2-dimethylpropylene, tetramethylene, hexamethylene, octamethylene, decamethylene or dodecamethylene.

If $R_4$ is $C_6$-$C_{12}$-arylene, it is for example o-, m- or p-phenylene, 1,4-naphthylene or 4,4'-diphenylene.

If $Z_1$ is $C_2$-$C_{12}$-alkanoyl, it is for example propionyl, butyryl, octanoyl, dodecanoyl or preferably acetyl.

As $C_5$-$C_7$cycloalkyl, $R_7$ is particularly cyclohexyl.

As $C_6$-$C_{10}$-aryl, $R_7$ is particularly phenyl or $\alpha$- or $\beta$-naphthyl which is unsubstituted or substituted with halogen or $C_1$-$C_4$-alkyl.

As $C_1$-$C_3$-alkylene, E is for example methylene, ethylene or propylene.

As $C_2$-$C_6$-alkylene, G is for example ethylene, propylene, 2,2-dimethylpropylene, tetramethylene or hexamethylene; and as $C_6$-$C_{12}$-arylene, G is o-, m- or p-phenylene, 1,4-naphthylene or 4,4'-diphenylene.

Diisocyanates of the formula $L_2$-$(NCO)_2$ useful to form the compounds of formula XVI or XVII are aliphatic, cycloaliphatic or aromatic diisocyanates and are selected from the group consisting of ethylene diisocyanate, 1,2-diisocyanatopropane, 1,3-diisocyanatopropane, 1,6-diisocyanatohexane, 1,2-diisocyanatocyclohexane, 1,3-diisocyanatocyclohexane, 1,4-diisocyanatobenzene, bis(4-isocyanatocyclohexyl)methane, bis(4-isocyanatocyclohexenyl)methane, bis(4-isocyanato-phenyl)methane, 2,6-and 2,4-toluene diisocyanate, 3,3-dichloro-4,4'-diisocyanatobiphenyl, 1,5-diisocyanatonaphthalene, hydrogenated toluene diisocyanate, 1-isocyanato-5-isocyanato 1,3,3-trimethylcyclohexane (= isophorone diisocyanate), 2,2'-diisocyanatodiethyl fumarate, 1,5-diisocyanato-1-carboxypentane, 1,2-, 1,3-, 1,6-, 1,7-, 2,7- and 2,3-diisocyanatonaphthalene, 2,4- and 2,7-diisocyanato-1-methylnaphthalene, 4,4'-diisocyanatobiphenyl, bis(4-isocyanatophenyl)ethane, bis(4-isocyanatophenyl) ether and 2,2,4-(2,4,4)-trimethylhexane-1,6-diisocyanate.

The preferred diisocyanates are isophorone diisocyanate, 2,2,4-(2,4,4)-trimethylhexane-1,6-diisocyanate and 2,4- and 2,6-toluene diisocyanate.

An effective inhibiting amount of an instant components (i) and (ii) of this invention needed to retard or prevent premature free radical induced polymerization of a monomer or monomer mixture is as follows: the weight ratio of component (i) to component (ii) is from 1:10 to 1000:1, preferably 1:1 to 10:1, and where the total concentration is in the range of 50-10,000 ppm, preferably 200-600 ppm, based on the monomer being stabilized. The lower amounts would be used where the degree of inhibition required is not great such as when the monomers are to be used promptly, or which will be stored refrigerated, or which are inherently less prone to polymerize readily such as monomers with internal double bonds. The higher amounts of inhibitor would be used where the monomer is to be stored for prolonged periods of time, especially under relatively warm conditions or where contamination is likely, or where exposure to photoinitiation is likely, or where the monomer is especially prone to rapid polymerization with little provocation such as with the acrylates and acrylic acid. Those skilled in the art of vinyl polymerization are well aware of the relative polymerizability of monomers and of their relative stabilities.

The stabilized compositions of this invention are distinguished by their lack of color.

The compositions of the instant invention may also contain additional inhibitors, such as hydroquinone, the monomethyl ether of hydroquinone, (these often being required by monomer specifications) or catechol, tert-butylated hydroquinones or catechols, other alkylated phenols, nitrosophenols and nitrosophenylhydroxylamines.

The inhibited compositions may also contain metal deactivators and UV absorbers to improve light stability; or stabilizers such as amines to retard acid-catalyzed degradation; or thermal or photoinitiators; and other conventional additives.

When it is desired to subject the inhibited monomer to polymerization, the inhibitor can either be removed or overridden by sufficient polymerization initiator. Removal can be accomplished by distillation, absorption or washing with an acidic solution. It is possible to remove the instant 1-hydroxy derivatives while leaving the phenolic antioxidants in the monomer by use of strong acid ion exchange resins. The polymerization inhibiting action of the instant compounds can be overridden by use of sufficient free radical initiator, actinic light irradiation, electron beam exposure or other polymerization initiating means.

The instant invention also pertains to a process for preventing the premature polymerization of a monomer polymerizable by free radical initiation which comprises adding to said monomer (a) an effective amount of a combination of compounds of any of the components (b) described above. The process of the instant invention involves simply dissolving an effective inhibiting amount of the inhibitor in the monomer prior to exposure of the latter to conditions where the premature, undesired free radical initiated polymerization might occur.

Preferably, this process comprises adding 50 to 10,000 of a mixture of components (i) and (ii) in a weight ratio of from 1:10 to 1000:1 to a continuous fluid feed stream to deactivate the autocatalytic polymerization, in any part of the continuous process equipment, such as reactor, reboiler, distillation column, etc., of any ethylenically unsaturated monomer present in the feed stream, and

further adding to said feed stream an additional 10 ppm to 500 ppm of said mixture as a makeup additive to maintain the desired concentration of said mixture in the fluid feed stream being processed.

Preferably, this process is also carried out to prevent the fouling of processing equipment including reactors, pipes, stills, distillation columns, cracking towers and heat transfer surfaces during the processing of a monomer polymerizable by free radical initiation.

The following examples are presented for the purpose of illustration only and are not to be construed as limiting the instant invention in any manner whatsoever.

The apparatus used in the following experiments is fabricated from an 80 mm OD and 3 mm thick glass tubing, 14 inches (35.6 cm) high, closed on one end and flared at the other end to fit a resin kettle top. The

kettle is equipped with a water condenser and nitrogen inlet tube. Except where noted, the polymer reported in the examples is formed in the vapor phase (refluxing) region on the walls of the apparatus about 3 inches (7.6 cm) above the surface of the liquid monomer.

Example 1: To demonstrate the need for a pot stabilizer, an experiment is run without any stabilizing additive in the acrylic acid monomer. A dry resin kettle is weighed and the weight recorded. A 100 ml (105 g) aliquot of acrylic acid is charged into the kettle and flushed with dry nitrogen flowing at 250 ml/min for 15 minutes. The kettle is then immersed in an oil bath containing approximately 6 liters of oil such that the surface of the acrylic acid in the kettle is about 2 inches (5.1 cm) below the level of the oil in the bath. The oil bath is heated to 150 °C and the acrylic acid is refluxed for 70 minutes. After approximately 15 minutes, the monomer begins to gel and white insoluble polymer is observed to grow in the monomer liquid. Only a small amount of acrylic acid monomer remains after 70 minutes. The kettle is removed from the oil bath and wiped free of oil. The resulting white polymer is rinsed with hexane to remove residual monomer, dried and weighed to determine the total amount of polymeric material collected inside the kettle. Some 82.9 g of white polymer is obtained. The resin kettle cannot be used for any further experiments.

Examples 3-24: Following the general procedure of Example 1, 100 ml (105 g) of acrylic acid refluxed for 70 minutes in the presence of the stabilizers indicated and the amount of polymer obtained is a measure of the relative effectiveness of the stabilizers use. The lesser is the amount of polymer formed, the more effective is the stabilizer used. The results are given in the table below.

| Example | Additive* (100 ppm) | Pot Stabilizer (1000 ppm) | Polymer Obtained (grams) |
|---|---|---|---|
| 3 | B | phenothiazine | 5.2 |
| 4 | C | phenothiazine | 22.5 |
| 5 | D | phenothiazine | 9.0 |
| 6 | E | phenothiazine | 1.6 |
| 7 | F | phenothiazine | 7.2 |
| 8 | G | phenothiazine | 7.2 |
| 9 | H | phenothiazine | 8.8 |
| 10 | I | phenothiazine | 9.0 |
| 11 | J | phenothiazine | 9.1 |
| 12 | K | phenothiazine | 12.6 |
| 13 | L | phenothiazine | 16.3 |
| 14 | M | phenothiazine | 18.4 |
| 15 | N | phenothiazine | 23.4 |
| 16 | O | phenothiazine | 29.3 |
| 17 | P | phenothiazine | 32.3 |
| 18 | Q | phenothiazine | 33.1 |
| 19 | R | phenothiazine | 40.5 |
| 20 | S | phenothiazine | 41.7 |
| 21 | T | phenothiazine | 44.1 |
| 11 | U | phenothiazine | 52.4 |
| 23 | V | phenothiazine | 56.5 |
| 24 | W | phenothiazine | 58.3 |

*

B is 1-[2-(methoxycarbonyl)ethoxy]-4-benzyloxy-2,2,6,6-tetramethyl piperidine.

C is 1-methoxy-2,2,6,6-tetramethylpiperidin-4-yl benzoate.

D is bis[1-(2-(methoxycarbonyl)ethoxy)-2,2,6,6-tetramethylpiperidi n-4-yl] phthalate.

E is 1-tert-butoxy-2,2,6,6-tetramethylpiperidin-4-yl benzoate.

F is 4-hydroxy-1-octyloxy-2,2,6,6-tetramethylpiperidine.

G is 1-[2-(methoxycarbonyl)ethoxy]-2,2,6,6-tetramethylpiperidine.

H is 1-methylcyclohexyloxy-2,2,6,6-tetramethylpiperidin-4-yl benzoate.

I is 4-benzyloxy-1-ethoxy-2,2,6,6-tetramethylpiperidine.

J is 1-carbamoyloxy-4-benzoyloxy-2,2,6,6-tetramethylpiperidine.

K is 4-hydroxy-1,2,2,6,6-pentamethylpiperidine.

L is 1-butylcarbamoyloxy-4-benzoyloxy-2,2,6,6-tetramethylpiperidin e.

M is 1-α-methylbenzyloxy-2,2,6,6-tetramethylpiperidin-4-yl benzoate.

N is 1,4-dimethoxy-2,2,6,6-tetramethylpiperidine.

O is bis[1-(2-(methoxycarbonyl)ethoxy)-2,2,6,6-tetramethylpiperidin-4-yloxy]-p-xylylene.

P is N,N-di-tert-butylhydroxylamine.

Q is 1-hydroxy-2,2,6,6-piperidin-4-yl benzoate.

R is 4-hydroxy-1-methoxy-2,2,6,6-tetramethylpiperidine.

S is 1-methoxy-2,2,6,6-tetramethylpiperidin-4-one.

T is 1-hydroxy-2,2,6,6-tetramethylpiperidin-4-one.

U is 4-hydroxyethoxy-2,2,6,6-tetramethylpiperidine.

V is bis(1-hydroxy-2,2,6,6-tetramethylpiperidin-4-yl) sebacate.

W is bis(1-octyloxy-2,2,6,6-tetramethylpiperidin-4-yl) sebacate.

Examples 25-27: When using the procedure of Example 3, the acrylic acid is replaced respectively with the monomers shown below, no polymer is formed in the presence of 100 ppm of 1-tert-butoxy-2,2,6,6-tetramethylpiperidin-4-yl benzoate and 1000 ppm of phenothiazine.

| Example | Monomer |
|---|---|
| 25 | methyl methacrylate |
| 26 | 2-hydroxyethyl methacrylate |
| 27 | 2-hydroxyethyl acrylate |

## Claims

**1.** A monomer composition, stabilized against premature polymerization, which comprises

(a) an ethylenically unsaturated monomer or mixture of monomers, polymerizable by free radical initiation, and

(b) an effective amount, sufficient to inhibit premature polymerization of component (a), of a combination of

(i) a heterocyclic compound selected from any of formulas A to D

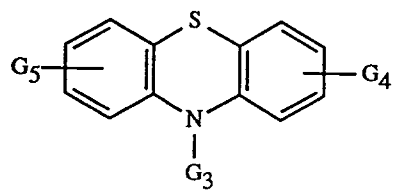

(A)

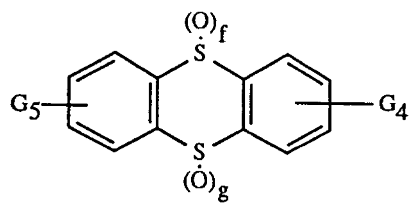

(B)

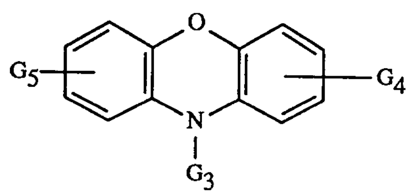

(C)

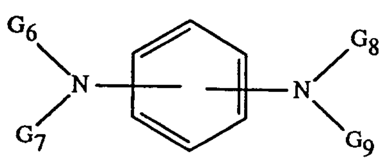

(D)

where
$G_3$ is hydrogen, alkyl of 1 to 4 carbon atoms or alkenyl of 3 to 4 carbon atoms,
$G_4$ and $G_5$ are independently hydrogen or alkyl of 1 to 8 carbon atoms,
$G_6$ is aryl of 6 to 10 carbon atoms or aralkyl of 7 to 15 carbon atoms, and
$G_7$, $G_8$ and $G_9$ are independently hydrogen, alkyl of 1 to 18 carbon atoms, cycloalkyl of 5 to 12 carbon atoms, aralkyl of 7 to 15 carbon atoms or alkaryl of 7 to 15 carbon atoms; f and g are independently 0, 1 or 2, and

(ii) a compound or mixture of compounds of any of formulas I to XIX

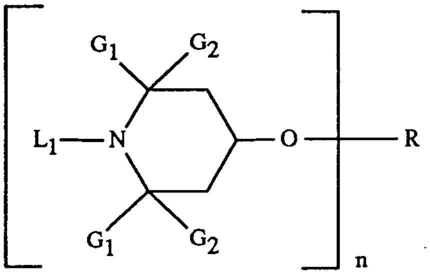

(I)

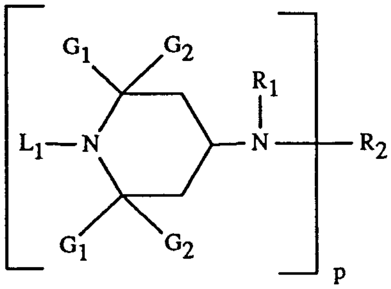

(II)

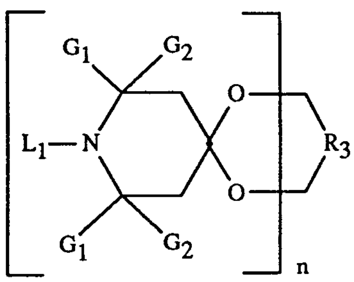

(III)

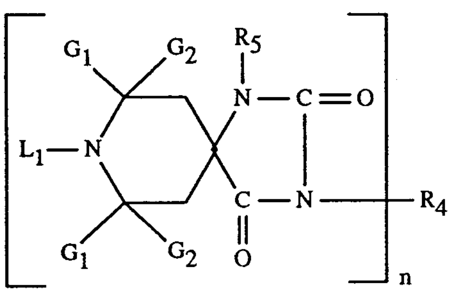

(IV)

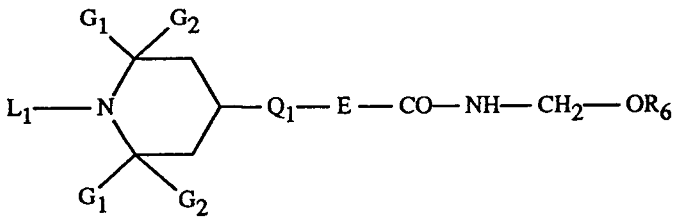

(V)

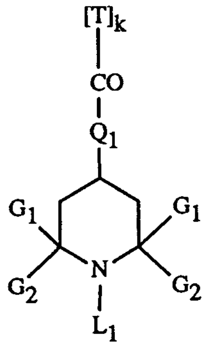

(VI)

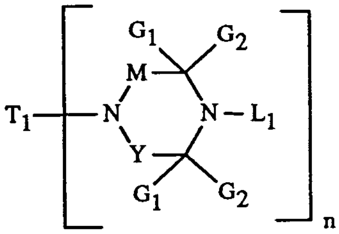

(VII)

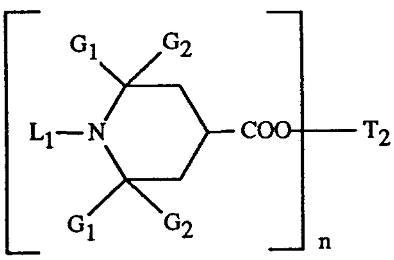

(VIII)

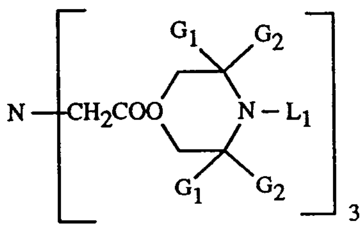

(IX)

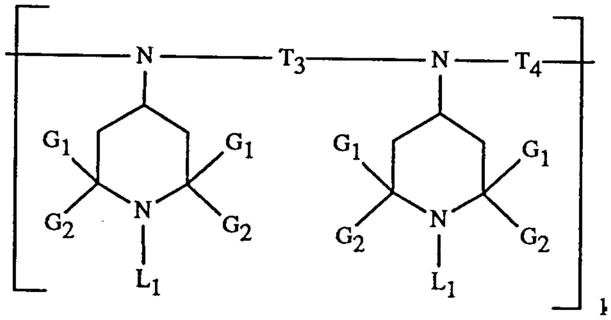

(X)

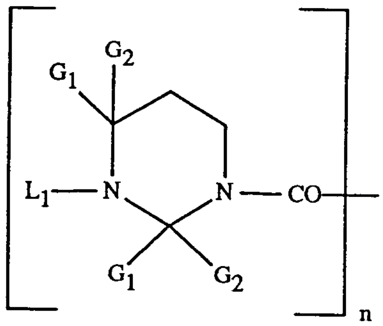

(XI)

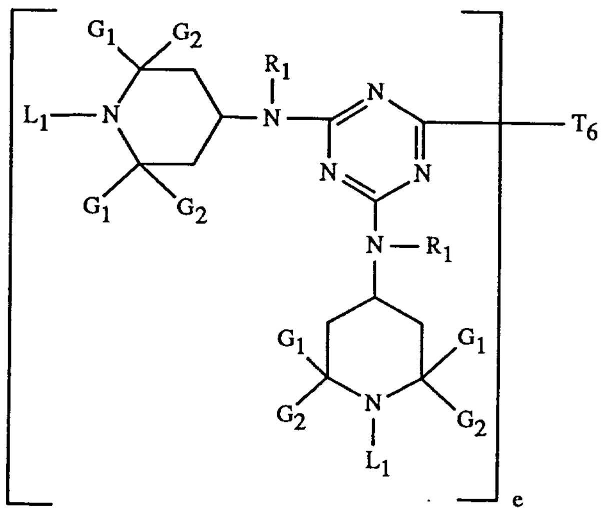

(XII)

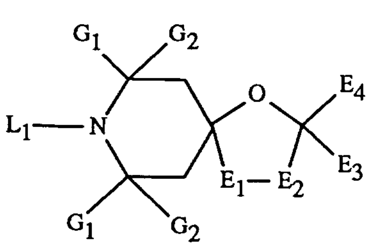

(XIII)

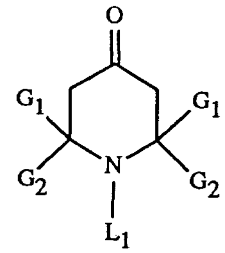

(XIV)

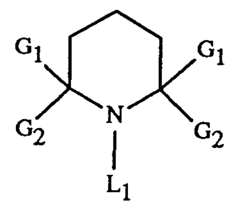

(XV)

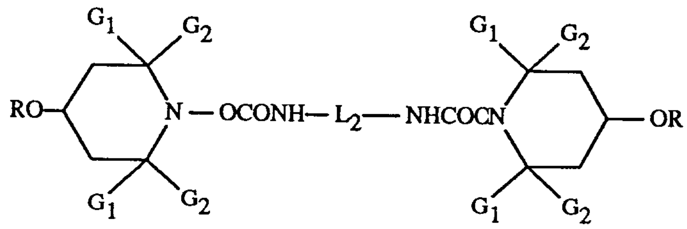

XVI

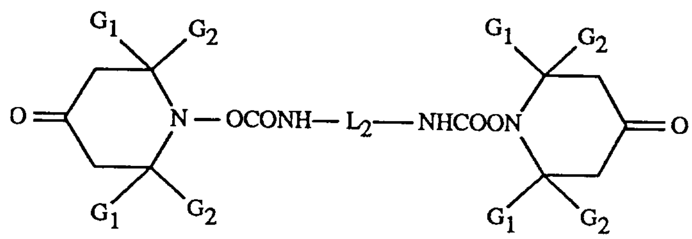

XVII

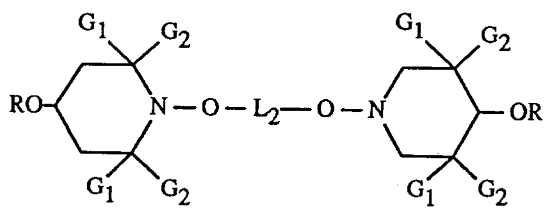

XVIII

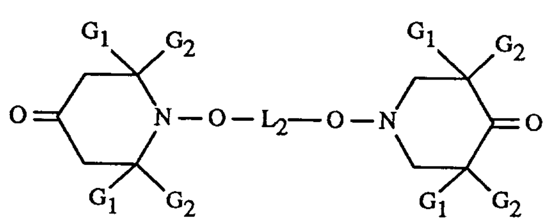

XIX

wherein

$G_1$ and $G_2$ are independently alkyl of 1 to 4 carbon atoms;

$L_1$ is hydrogen, hydroxyl, alkyl of 1 to 18 carbon atoms, said alkyl substituted by hydroxyl, cyanoethyl, glycidyl, aralkyl of 7 to 15 carbon atoms or a monovalent acyl radical of an aliphatic, cycloaliphatic, araliphatic or aromatic acid, $-OCONHL_3$ or -OL4, where

$L_3$ is hydrogen, alkyl of 2 to 18 carbon atoms, allyl, cyclohexyl, aryl of 6 to 10 carbon atoms, said aryl substituted by one or two alkyl groups of 1 to 4 carbon atoms or is benzyl,

$L_4$ is alkyl of 1 to 18 carbon atoms, cycloalkyl of 5 to 12 carbon atoms, alkenyl of 2 to 18 carbon atoms, cycloalkenyl of 5 to 12 carbon atoms, aralkyl of 7 to 15 carbon atoms, a radical of a saturated or unsaturated bicyclic or tricyclic hydrocarbon of 7 to 12 carbon atoms or aryl of 6 to 10 carbon atoms or said aryl substituted by alkyl of 1 to 4 carbon atoms, or $L_4$ is $-CH_2CH_2COOL_5$ where $L_5$ is alkyl of 1 to 18 carbon atoms,
n is 1 or 2,
when n is 1,
R is hydrogen, $C_1$-$C_{18}$-alkyl optionally interrupted by one or more oxygen atoms, cyanoethyl, benzyl, glycidyl, a monovalent acyl radical of an aliphatic, cycloaliphatic, araliphatic or aromatic acid, or of carbamic acid or of a phosphorus-containing acid, or a monovalent silyl radical; or
when n is 2,
R is $C_1$-$C_{12}$-alkylene, $C_4$-$C_{12}$-alkenylene, xylylene, a divalent acyl radical of an aliphatic, cycloaliphatic, araliphatic or aromatic dicarboxylic acid, or of a dicarbamic acid or of a phosphorus-containing acid, or a bivalent silyl radical;
p is 1, 2 or 3,
$R_1$ is $C_1$-$C_{12}$-alkyl, $C_5$-$C_7$-cycloalkyl, $C_7$-$C_8$-aralkyl, $C_2$-$C_{18}$-alkanoyl, $C_3$-$C_5$-alkenoyl or benzoyl;
when p is 1,
$R_2$ is $C_1$-$C_{18}$-alkyl, $C_5$-$C_7$-cycloalkyl, $C_2$-$C_8$-alkenyl unsubstituted or substituted by a cyano, carbonyl or carbamide group, or is glycidyl, a group of the formula
$-CH_2CH(OH)-Z$ or of the formula -CONH-Z wherein Z is hydrogen, methyl or phenyl; or when p is 2,
$R_2$ is $C_2$-$C_{12}$-alkylene, $C_6$-$C_{12}$-arylene, xylylene, a
$-CH_2CH(OH)CH_2-O-X-O-CH_2CH(OH)CH_2-$ wherein X is $C_2$-$C_{10}$-alkylene , $C_6$-$C_{15}$-arylene or $C_6$-$C_{12}$-cycloalkylene; or, provided that $R_1$ is not alkanoyl, alkenoyl or benzoyl, $R_2$ can also be a divalent acyl radical of an aliphatic, cycloaliphatic or aromatic dicarboxylic acid or dicarbamic acid, or can be the group -CO-; or $R_1$ and $R_2$ together when p is 1 can be the cyclic acyl radical of an aliphatic or aromatic 1,2- or 1,3-dicarboxylic acid; or
$R_2$ is

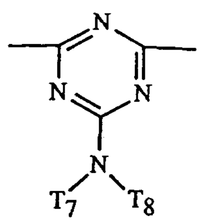

where $T_7$ and $T_8$ are independently hydrogen, alkyl of 1 to 18 carbon atoms, or $T_7$ and $T_8$ together are alkylene of 4 to 6 carbon atoms or 3-oxapentamethylene;
when p is 3,
$R_2$ is 2,4,6-triazinyl;
when n is 1,
$R_3$ is $C_2$-$C_8$-alkylene or hydroxyalkylene or $C_4$-$C_{22}$-acyloxyalkylene; or
when n is 2,
$R_3$ is $(-CH_2)_2C(CH_2-)_2$;
when n is 1,
$R_4$ is hydrogen, $C_1$-$C_{12}$-alkyl, $C_3$-$C_5$-alkenyl, $C_7$-$C_9$-aralkyl, $C_5$-$C_7$-cycloalkyl, $C_2$-$C_4$-hydroxyalkyl, $C_2$-$C_6$-alkoxyalkyl, $C_6$-$C_{10}$-aryl, glycidyl, a group of formula $-(CH_2)_m-COO-Q$ or of the formula $(CH_2)_m-O-CO-Q$ wherein m is 1 or 2 and Q is $C_1$-$C_4$-alkyl or phenyl; or
when n is 2,
$R_4$ is $C_2$-$C_{12}$-alkylene, $C_6$-$C_{12}$-arylene, a group
$-CH_2CH(OH)CH_2-O-X-O-CH_2CH(OH)CH_2-$ wherein X is $C_2$-$C_{10}$-alkylene, $C_6$-$C_{15}$-arylene or $C_6$-$C_{12}$-cycloalkylene, or a group
$-CH_2CH(OZ_1)CH_2-(OCH_2CH(OZ_1)CH_2)_2-$ wherein $Z_1$ is hydrogen, $C_1$-$C_{18}$-alkyl, allyl, benzyl, $C_2$-$C_{12}$-alkanoyl or benzoyl;
$R_5$ is hydrogen, $C_1$-$C_{12}$-alkyl, allyl, benzyl, glycidyl or $C_2$-$C_6$-alkoxyalkyl;
$Q_1$ is $-N(R_7)-$ or -O-;

E is $C_1$-$C_3$-alkylene, the group $-CH_2CH(R_8)-O-$ wherein $R_8$ is hydrogen, methyl or phenyl, the group $-(CH_2)_3-NH-$ or a direct bond;
$R_7$ is $C_1$-$C_{18}$-alkyl, $C_5$-$C_7$-cycloalkyl, $C_7$-$C_{12}$-aralkyl, cyanoethyl, $C_6$-$C_{10}$-aryl, the group $-CH_2CH(R_8)-OH$; or a group of the formula

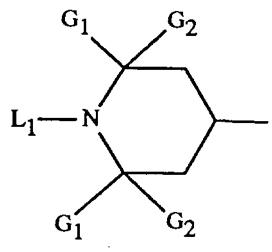

or a group of the formula

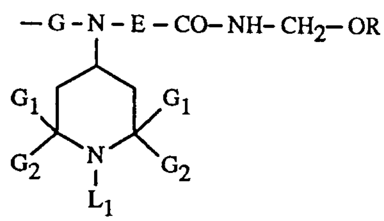

wherein G is $C_2$-$C_6$-alkylene or $C_6$-$C_{12}$-arylene; or
$R_7$ is a group $-E-CO-NH-CH_2-OR_6$
$R_6$ is hydrogen or $C_1$-$C_{18}$-alkyl;
Formula VI denotes a recurring structural unit of a polymer where T is ethylene or 1,2-propylene, or is a repeating structural unit derived from an $\alpha$-olefin copolymer with an alkyl acrylate or methacrylate;
k is 2 to 100;
$T_1$ has the same meaning as $R_2$ when p is 1 or 2;
M and Y are independently methylene or carbonyl;
$T_2$ has the same meaning as $R_4$, and $T_2$ is octamethylene;
$T_3$ and $T_4$ are independently alkylene of 2 to 12 carbon atoms, of $T_4$ is

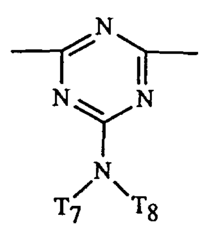

$T_6$ is

$$-NH(CH_2)_a-N(CH_2)_b-N[(CH_2)_c-N-]_dH$$

where a, b and c are independently 2 or 3, and d is 0 or 1;
e is 3 or 4;
$T_5$ is the same as R with the proviso that $T_5$ cannot be hydrogen when n is 1;
$E_1$ and $E_2$, being different, are each oxo or imino;
$E_3$ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms,

or said phenylalkyl substituted by alkyl of 1 to 4 carbon atoms; and
$E_4$ is hydrogen, alkyl of 1 to 30 carbon atoms, phenyl, naphthyl or phenylalkyl of 7 to 12 carbon atoms; or
$E_3$ and $E_4$ together are polymethylene of 4 to 17 carbon atoms, or said polymethylene substituted by up to four alkyl groups of 1 to 4 carbon atoms;
$L_2$ is a divalent radical from an aliphatic, cycloaliphatic or aromatic diisocyanate from which the two -NCO groups are removed, or is an alkanediyl of 1 to 18 carbon atoms or cyclohexanediyl

**2.** A composition according to claim 1 wherein the effective amount is a combination of component (i) and component (ii) in a weight ratio of (i):(ii) of 1:10 to 1000:1, preferably 1:1 to 10:1, and where the total concentration of (i) plus (ii) is in the range of 50-10,000 ppm, preferably 200-600 ppm, preferably 200-600 ppm, based on the monomer being stabilized.

**3.** A composition according to claim 1 wherein component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

**4.** A composition according to claim 3 wherein the monomer is styrene, butadiene, vinyl chloride, acrylic acid, methacrylic acid, vinyl acetate, 2-hydroxyethyl acrylate, 2-hydroxyethyl methacrylate, trimethylolpropane triacrylate, polyethylene glycol diacrylate or methyl methacrylate, preferably styrene, butadiene, acrylic acid or methacrylic acid.

**5.** A composition according to claim 1 wherein component (i) is phenothiazine.

**6.** A composition according to claim 1 wherein component (ii) is of formula I or XV, preferably of formula I.

**7.** A composition according to claim 1 wherein component (ii) is selected from the group consisting of
1-[2-(methoxycarbonyl)ethoxy]-4-benzyloxy-2,2,6,6-tetramethyl piperidine;
1-methoxy-2,2,6,6-tetramethylpiperidin-4-yl benzoate;
bis[1-(2-(methoxycarbonyl)ethoxy)-2,2,6,6-tetramethylpiperidi n-4-yl] phthalate;
1-tert-butoxy-2,2,6,6-tetramethylpiperidin-4-yl benzoate;
4-hydroxy-1-octyloxy-2,2,6,6-tetramethylpiperidine;
1-[2-(methoxycarbonyl)ethoxy]-2,2,6,6-tetramethylpiperidine; 1
-methylcyclohexyloxy-2,2,6,6-tetramethylpiperidin-4-yl benzoate;
4-benzyloxy-1-ethoxy-2,2,6,6-tetramethylpiperidine;
1-carbamoyloxy-4-benzoyloxy-2,2,6,6-tetramethylpiperidine;
4-hydroxy-1,2,2,6,6-pentamethylpiperidine;
1-butylcarbamoyloxy-4-benzoyloxy-2,2,6,6-tetramethylpiperidin e;
1-$\alpha$-methylbenzyloxy-2,2,6,6-tetramethylpiperidin-4-yl benzoate;
1,4-dimethoxy-2,2,6,6-tetramethylpiperidine;
bis[1-(2-(methoxycarbonyl)ethoxy)-2,2,6,6-tetramethylpiperidi n-4-yloxy]-p-xylylene;
1-hydioxy-2,2,6,6-piperidin-4-yl benzoate;
4-hydroxy-1-methoxy-2,2,6,6-tetramethylpiperidine;
1-methoxy-2,2,6,6-tetramethylpiperidin-4-one;
1-hydroxy-2,2,6,6-tetramethylpiperidin-4-one;
4-hydroxyethoxy-2,2,6,6-tetramethylpiperidine;
bis(1-hydroxy-2,2,6,6-tetramethylpiperidin-4-yl) sebacate; and
bis(1-octyloxy-2,2,6,6-tetramethylpiperidin-4-yl) sebacate.

**8.** A composition according to claim 1 which additionally contains another stabilizer selected from the group consisting of hydroquinone and monomethyl ether of hydroquinone.

**9.** A process for preventing the premature polymerization of a monomer polymerizable by free radical initiation which comprises
adding to said monomer (a) an effective amount of a combination of compounds of component (b) according to claim 1.

10. A process according to claim 9 for preventing the fouling of processing equipment including reactors, pipes, stills, distillation columns, cracking towers and heat transfer surfaces during the processing of a monomer polymerizable by free radical intiation which comprises
adding to said monomer, before processing is begun, an effective amount of a combination of compounds of component (b) according to claim 1.

11. A process according to claim 9 which comprises
adding 50 to 10,000 ppm of a mixture of components (i) and (ii), according to claim 13, in a weight ratio of from 1:10 to 1000:1, to a continuous feed stream to deactivate the autocatalytic polymerization, in any part of the continuous process equipment, of any ethylenically unsaturated monomer present in the feed stream, and
further adding to said feed stream an additional 10 ppm to 500 ppm of said mixture as a makeup additive to maintain the desired concentration of said mixture in the fluid feed stream being processed.

## Patentansprüche

1. Gegenüber einer vorzeitigen Polymerisation stabilisierte Monomerzusammensetzung, enthaltend
(a) ein ethylenisch ungesättigtes Monomeres oder Monomerengemisch, polymerisierbar durch Initiierung mit freien Radikalen, und
(b) eine wirksame Menge, die ausreichend ist für die Inhibierung einer vorzeitigen Polymerisation von Komponente (a), einer Kombination von
(i) einer heterocyclischen Verbindung, ausgewählt unter jedweder der Formeln A bis D

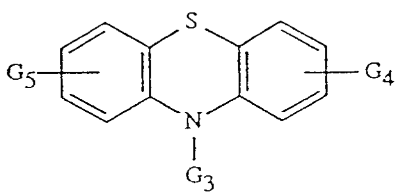

(A)

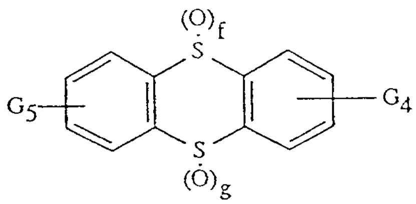

(B)

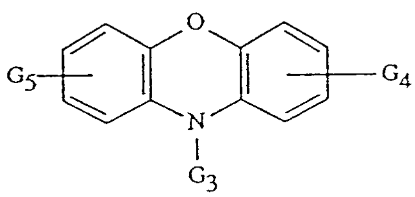

(C)

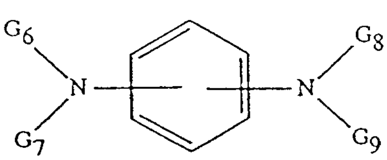

(D)

worin
$G_3$ für Wasserstoff, Alkyl mit 1 bis 4 Kohlenstoffatomen oder Alkenyl mit 3 bis 4 Kohlenstoffatomen steht,
$G_4$ und $G_5$ unabhängig Wasserstoff oder Alkyl mit 1 bis 8 Kohlenstoffatomen bedeuten,
$G_6$ für Aryl mit 6 bis 10 Kohlenstoffatomen oder Aralkyl mit 7 bis 15 Kohlenstoffatomen steht und
$G_7$, $G_8$ und $G_9$ unabhängig Wasserstoff, Alkyl mit 1 bis 18 Kohlenstoffatomen, Cycloalkyl mit 5

bis 12 Kohlenstoffatomen, Aralkyl mit 7 bis 15 Kohlenstoffatomen oder Alkaryl mit 7 bis 15 Kohlenstoffatomen bedeuten, f und g unabhängig für 0, 1 oder 2 stehen, und

(ii) einer Verbindung oder einem Gemisch von Verbindungen jedweder der Formeln I bis XIX

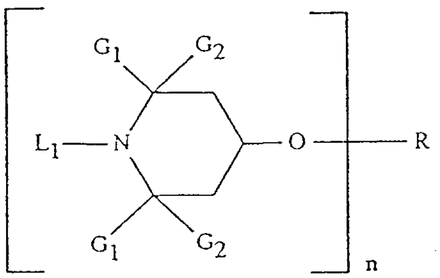

(I)

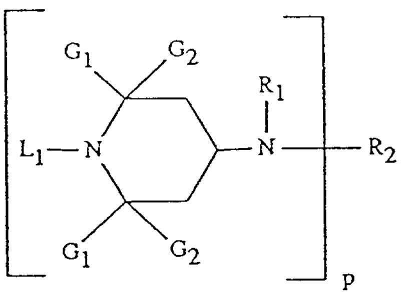

(II)

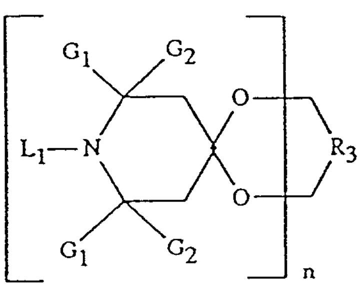

(III)

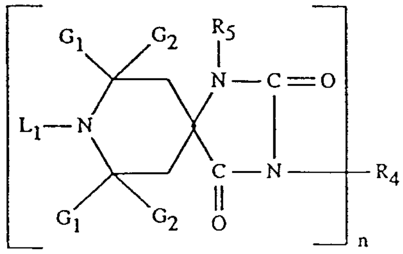

(IV)

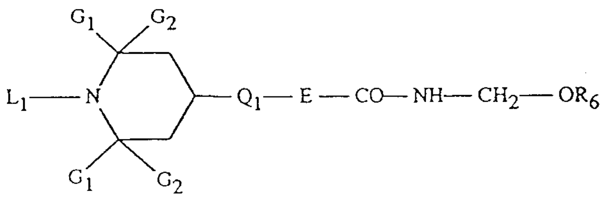

(V)

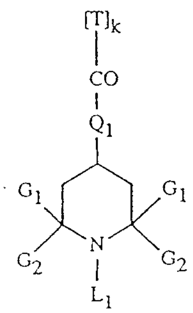

(VI)

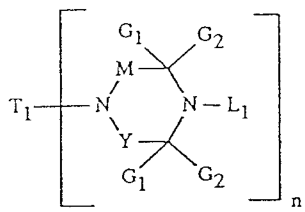

(VII)

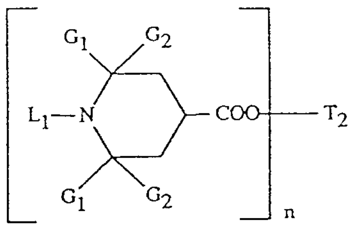

(VIII)

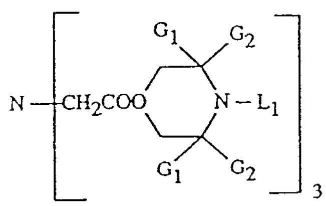

(IX)

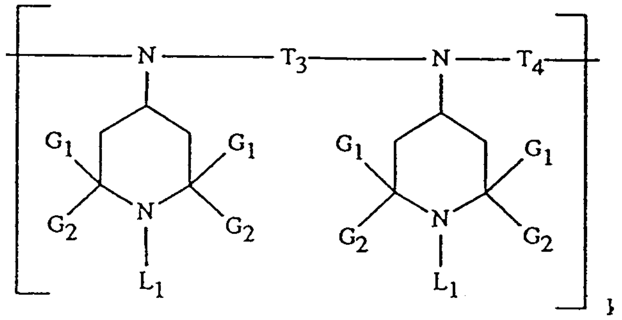

(X).

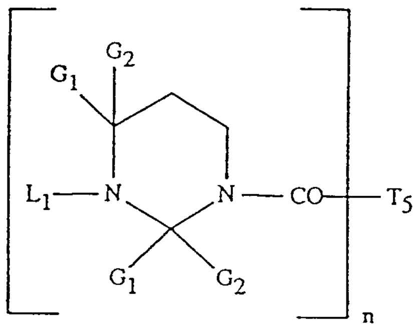

(XI)

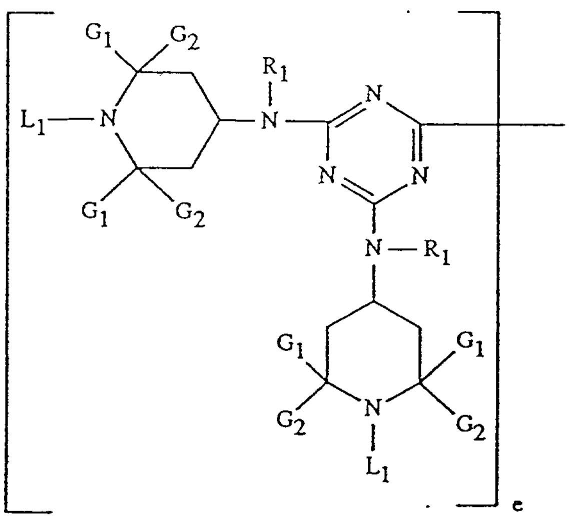

(XII)

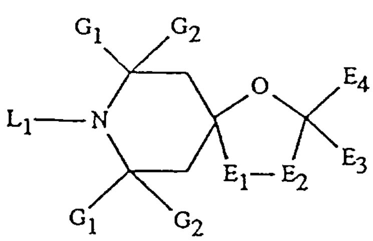

(XIII)

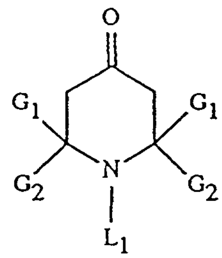

(XIV)

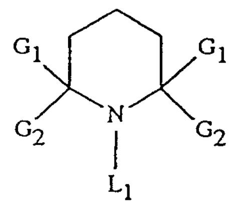

(XV)

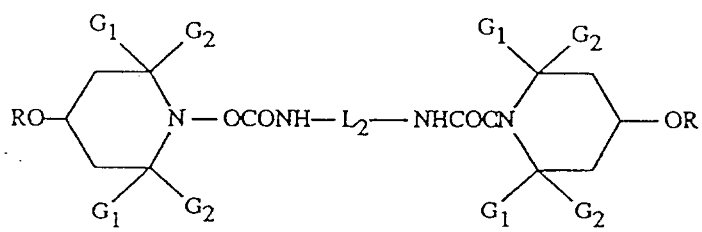

XVI

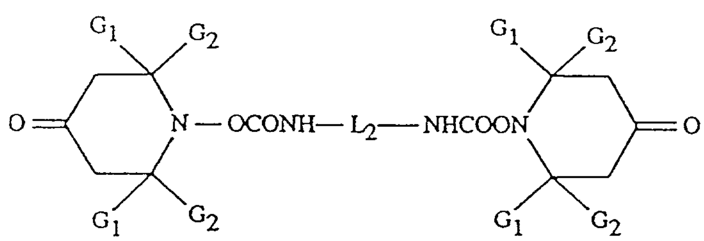

XVII

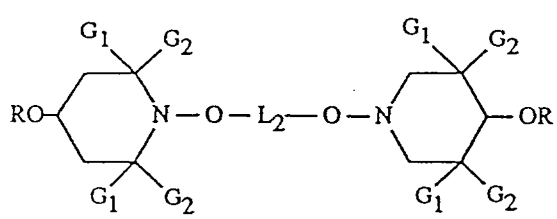

XVIII

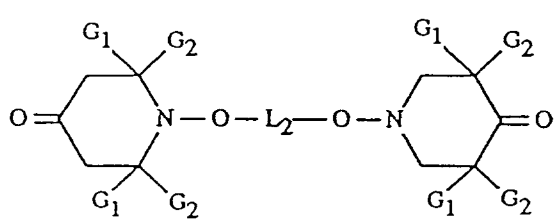

XIX

worin
$G_1$ und $G_2$ unabhängig für Alkyl mit 1 bis 4 Kohlenstoffatomen stehen,
$L_1$ Wasserstoff, Hydroxyl, Alkyl mit 1 bis 18 Kohlenstoffatomen, dieses Alkyl substituiert durch Hydroxyl, Cyanoethyl, Glycidyl, Aralkyl mit 7 bis 15 Kohlenstoffatomen oder einen einwertigen Acylrest einer aliphatischen, cycloaliphatischen, araliphatischen oder aromatischen Säure, $-OCONHL_3$ oder $-OL_4$ bedeutet, wobei $L_3$ Wasserstoff, Alkyl mit 2 bis 18 Kohlenstoffatomen, Allyl,

Cyclohexyl, Aryl mit 6 bis 10 Kohlenstoffatomen, dieses Aryl substituiert durch eine oder zwei Alkylgruppen mit 1 bis 4 Kohlenstoffatomen, oder Benzyl bedeutet,
$L_4$ Alkyl mit 1 bis 18 Kohlenstoffatomen, Cycloalkyl mit 5 bis 12 Kohlenstoffatomen, Alkenyl mit 2 bis 18 Kohlenstoffatomen, Cycloalkenyl mit 5 bis 12 Kohlenstoffatomen, Aralkyl mit 7 bis 15 Kohlenstoffatomen, einen Rest eines gesättigten oder ungesättigten, bicyclischen oder tricyclischen Kohlenwasserstoffs mit 7 bis 12 Kohlenstoffatomen oder Aryl mit 6 bis 10 Kohlenstoffatomen oder dieses Aryl, substituiert durch Alkyl mit 1 bis 4 Kohlenstoffatomen, bedeutet oder $L_4$ für $-CH_2CH_2COOL_5$ steht, wobei $L_5$ Alkyl mit 1 bis 18 Kohlenstoffatomen bedeutet,
n für 1 oder 2 steht,
wenn n für 1 steht,
R Wasserstoff, $C_{1-18}$-Alkyl, gegebenenfalls unterbrochen durch ein oder mehrere Sauerstoffatome, Cyanoethyl, Benzyl, Glycidyl, einen einwertigen Acylrest einer aliphatischen, cycloaliphatischen, araliphatischen oder aromatischen Säure oder einer Carbamidsäure oder einer phosphorhaltigen Säure oder einen einwertigen Silylrest bedeutet; oder
wenn n für 2 steht,
R $C_{1-12}$-Alkylen, $C_{4-12}$-Alkenylen, Xylylen, einen zweiwertigen Acylrest einer aliphatischen, cycloaliphatischen, araliphatischen oder aromatischen Dicarbonsäure oder einer Dicarbamidsäure oder einer phosphorhaltigen Säure oder einen zweiwertigen Silylrest bedeutet;
p für 1, 2 oder 3 steht,
$R_1$ $C_{1-12}$-Alkyl, $C_{5-7}$-Cycloalkyl, $C_{7-8}$-Aralkyl, $C_{2-18}$-Alkanoyl, $C_{3-5}$-Alkenoyl oder Benzoyl ist;
wenn p für 1 steht,
$R_2$ $C_{1-18}$-Alkyl, $C_{5-7}$-Cycloalkyl, $C_{2-8}$-Alkenyl, unsubstituiert oder substituiert durch eine Cyano-, Carbonyl- oder Carbamidgruppe, ist oder Glycidyl, eine Gruppe der Formel $-CH_2CH(OH)-Z$ oder der Formel -CONH-Z ist, wobei Z für Wasserstoff, Methyl oder Phenyl steht; oder
wenn p für 2 steht,
$R_2$ $C_{2-12}$-Alkylen, $C_{6-12}$-Arylen, Xylylen, eine Gruppe $-CH_2CH(OH)-CH_2-O-X-O-CH_2CH(OH)CH_2-$ bedeutet, worin X für $C_{2-10}$-Alkylen, $C_{6-15}$-Arylen oder $C_{6-12}$-Cycloalkylen steht; oder mit der Maßgabe, daß $R_1$ nicht Alkanoyl, Alkenoyl oder Benzoyl ist, $R_2$ auch ein zweiwertiger Acylrest einer aliphatischen, cycloaliphatischen oder aromatischen Dicarbonsäure oder Dicarbamidsäure sein kann oder die Gruppe -CO- sein kann; oder $R_1$ und $R_2$ gemeinsam, wenn p für 1 steht, der cyclische Acylrest einer aliphatischen oder aromatischen 1,2- oder 1,3-Dicarbonsäure sein können; oder
$R_2$ für

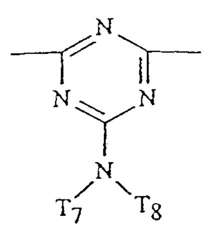

worin $T_7$ und $T_8$ unabhängig Wasserstoff, Alkyl mit 1 bis 18 Kohlenstoffatomen bedeuten oder $T_7$ und $T_8$ gemeinsam Allen mit 4 bis 6 Kohlenstoffatomen oder 3-Oxapentamethylen darstellen, steht,
wenn p für 3 steht,
$R_2$ 2,4,6-Triazinyl ist;
wenn n für 1 steht,
$R_3$ $C_{2-8}$-Alkylen oder Hydroxyalkylen oder $C_{4-22}$-Acyloxyalkylen ist; oder
wenn n für 2 steht,
$R_3$ $(-CH_2)_2C(CH_2-)_2$ ist;
wenn n für 1 steht,
$R_4$ Wasserstoff, $C_{1-12}$-Alyl, $C_{3-5}$-Alkenyl, $C_{7-9}$-Aralkyl, $C_{5-7}$-Cycloalkyl, $C_{2-4}$-Hydroxyalkyl, $C_{2-6}$-Alkoxyalkyl, $C_{6-10}$-Aryl, Glycidyl, eine Gruppe der Formel $-(CH_2)_m-COO-Q$ oder der Formel $-(CH_2)_m-O-CO-Q$ bedeutet, wobei m für 1 oder 2 steht und Q $C_{1-4}$-Alkyl oder Phenyl ist; oder
wenn n für 2 steht,
$R_4$ $C_{2-12}$-Alkylen, $C_{6-12}$-Arylen, eine Gruppe $-CH_2CH(OH)CH_2-O-X-O-CH_2CH(OH)CH_2-$ wobei X

für $C_{2-10}$-Alkylen, $C_{6-15}$-Arylen oder $C_{6-12}$-Cycloalkylen steht, oder eine Gruppe $-CH_2CH(OZ_1)-CH_2-(OCH_2CH(OZ_1)CH_2)_2$, worin $Z_1$ für Wasserstoff, $C_{1-18}$-Alkyl, Allyl, Benzyl, $C_{2-12}$-Alkanoyl oder Benzoyl steht, bedeutet;

$R_5$ für Wasserstoff $C_{1-12}$-Alkyl, Allyl, Benzyl, Glycidyl oder $C_{2-6}$-Alkoxyalkyl steht;

$Q_1$ $-N(R_7)-$ oder -O- bedeutet;

E $C_{1-3}$-Alkylen, die Gruppe $-CH_2CH(R_8)-O-$, wobei $R_8$ für Wasserstoff, Methyl oder Phenyl steht, die Gruppe $-(CH_2)_3-NH-$ oder eine direkte Bindung ist;

$R_7$ $C_{1-18}$-Alkyl, $C_{5-7}$-Cycloalkyl, $C_{7-12}$-Aralkyl, Cyanoethyl, $C_{6-10}$-Aryl, die Gruppe $-CH_2CH(R_8)-OH$ oder eine Gruppe der Formel

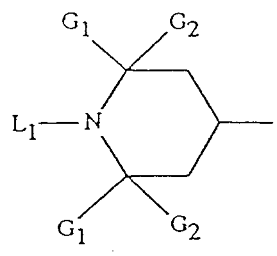

oder eine Gruppe der Formel

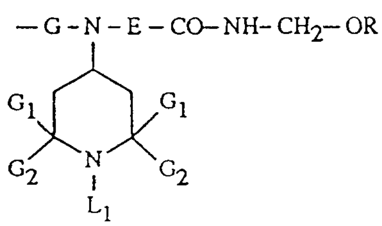

worin G für $C_{2-6}$-Alkylen oder $C_{6-12}$-Arylen steht, bedeutet;

oder

$R_7$ eine Gruppe $-E-CO-NH-CH_2-OR_6$ ist;

$R_6$ für Wasserstoff oder $C_{1-18}$-Alkyl steht;

die Formel VI eine wiederkehrende Struktureinheit eines Polymeren bezeichnet, worin T Ethylen oder 1,2-Propylen bedeutet, oder eine wiederkehrende Struktureinheit, abgeleitet von einem α-Olefincopolymeren mit einem Alkylacrylat oder -methacrylat, ist;

k für 2 bis 100 steht;

$T_1$ die gleiche Bedeutung wie $R_2$ besitzt, wenn p für 1 oder 2 steht;

M und Y unabhängig Methylen oder Carbonyl bedeuten;

$T_2$ die gleiche Bedeutung wie $R_4$ besitzt und $T_2$ Octamethylen ist;

$T_3$ und $T_4$ unabhängig Alkylen mit 2 bis 12 Kohlenstoffatomen bedeuten oder $T_4$

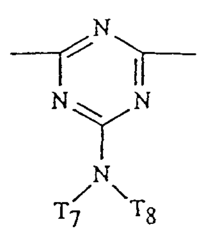

ist;

$T_6$ für

$$-NH(CH_2)_a-N(CH_2)_b-N[(CH_2)_c-N-]_dH$$

steht,
worin a, b und c unabhängig für 2 oder 3 stehen und d für 0 oder 1 steht;
e für 3 oder 4 steht;
$T_5$ die gleiche Bedeutung wie R besitzt, mit der Maßgabe, daß $T_5$ nicht Wasserstoff sein kann, wenn n für 1 steht;
$E_1$ und $E_2$ verschieden sind und jeweils Oxo oder Imino bedeuten;
$E_3$ Wasserstoff, Alkyl mit 1 bis 30 Kohlenstoffatomen, Phenyl, Naphthyl, dieses Phenyl oder dieses Naphthyl substituiert durch Chlor oder durch Alkyl mit 1 bis 4 Kohlenstoffatomen, oder Phenylalkyl mit 7 bis 12 Kohlenstoffatomen oder dieses Phenylalkyl substituiert durch Alkyl mit 1 bis 4 Kohlenstoffatomen, bedeutet; und
$E_4$ Wasserstoff, Alkyl mit 1 bis 30 Kohlenstoffatomen, Phenyl, Naphthyl oder Phenylalkyl mit 7 bis 12 Kohlenstoffatomen bedeutet; oder
$E_3$ und $E_4$ gemeinsam Polymethylen mit 4 bis 17 Kohlenstoffatomen oder dieses Polymethylen, substituiert mit bis zu 4 Alkylgruppen mit 1 bis 4 Kohlenstoffatomen, sind;
$L_2$ ein zweiwertiger Rest aus einem aliphatischen, cycloaliphatischen oder aromatischen Diisocyanat ist, aus dem die beiden -NCO-Gruppen entfernt sind, oder ein Alkandiyl mit 1 bis 18 Kohlenstoffatomen oder Cyclohexandiyl ist.

**2.** Zusammensetzung gemäß Anspruch 1, worin die wirksame Menge eine Kombination der Komponente (i) und Komponente (ii) in einem Gewichtsverhältnis von (i):(ii) von 1:10 bis 1000:1, vorzugsweise 1:1 bis 10:1 ist und worin die Gesamtkonzentration von (i) plus (ii) im Bereich von 50 bis 10 000 ppm, vorzugsweise 200 bis 600 ppm, bevorzugt 200 bis 600 ppm, basierend auf dem zu stabilisierenden Monomeren, beträgt.

**3.** Zusammensetzung gemäß Anspruch 1, worin die Komponente (a) ein Monomeres, ausgewählt unter den olefinischen Kohlenwasserstoffen, Dienen, halogenierten Monomeren, ungesättigten Säuren, ungesättigten Estern, ungesättigten Amiden, ungesättigten Nitrilen, ungesättigten Ethern, acrylierten Urethanen und ungesättigten Polyestern und Mischungen hiervon ist.

**4.** Zusammensetzung gemäß Anspruch 3, worin das Monomere Styrol, Butadien, Vinylchlorid, Acrylsäure, Methacrylsäure, Vinylacetat, 2-Hydroxyethylacrylat, 2-Hydroxyethylmethacrylat, Trimethylolpropan-triacrylat, Polyethylenglykol-diacrylat oder Methylmethacrylat, vorzugsweise Styrol, Butadien, Acrylsäure oder Methacrylsäure, ist.

**5.** Zusammensetzung gemäß Anspruch 1, worin die Komponete (i) Phenothiazin ist.

**6.** Zusammensetzung gemäß Anspruch 1, worin die Komponente (ii) die Formel I oder XV, vorzugsweise die Formel I, besitzt.

**7.** Zusammensetzung gemäß Anspruch 1, worin die Komponente (ii) aus der folgenden Gruppe ausgewählt ist:
1-[2-(Methoxycarbonyl)-ethoxy]-4-benzyloxy-2,2,6,6-tetramemethyl-piperidin;
1-Methoxy-2,2,6,6-tetramethylpiperidin-4-yl-benzoat;
Bis-[1-(2-(methoxycarbonyl)-ethoxy)-2,2,6,6-tetramethylpiperidin-4-yl]-phthalat;
1-tert.-Butoxy-2,2,6,6-tetramethylpiperidin-4-yl-benzoat;
4-Hydroxy-1-octyloxy-2,2,6,6-tetramethylpiperidin;
1-[2-(Methoxycarbonyl)-ethoxy]-2,2,6,6-tetramethylpiperidin;
1-Methylcyclohexyloxy-2,2,6,6-tetramethylpiperidin-4-yl-benzoat;
4-Benzyloxy-1-ethoxy-2,2,6,6-tetramethylpiperidin;
1-Carbamoyloxy-4-benzoyloxy-2,2,6,6-tetramethylpiperidin;
4-Hydroxy-1,2,2,6,6-pentamethylpiperidin;
1-Butylcarbamoyloxy-4-benzoyloxy-2,2,6,6-tetramethylpiperidin;
1-$\alpha$-Methylbenzyloxy-2,2,6,6-tetramethylpiperidin-4-yl-benzoat;
1,4-Dimethoxy-2,2,6,6-tetramethylpiperidin;

Bis-[1-(2-(methoxycarbonyl)-ethoxy)-2,2,6,6-tetramethylpiperidin-4-yl-oxy]-p-xylylen;
1-Hydroxy-2,2,6,6-piperidin-4-yl-benzoat;
4-Hydroxy-1-methoy-2,2,6,6-tetramethylpiperidin;
1-Methoxy-2,2,6,6-tetramethylpiperidin-4-on;
1-Hydroxy-2,2,6,6-tetramethylpiperidin-4-on;
4-Hydroxyethoxy-2,2,6,6-tetramethylpiperidin;
Bis-(1-hydroxy-2,2,6,6-tetramethylpiperidin-4-yl)-sebacat;
und
Bis-(1-octyloxy-2,2,6,6-tetramethylpiperidin-4-yl)-sebacat.

**8.** Zusammensetzung gemäß Anspruch 1, die zusätzlich einen weiteren Stabilisator, ausgewählt unter Hydrochinon und Hydrochinon-monomethylether, enthält.

**9.** Verfahren zur Verhinderung einer vorzeitigen Polymerisation eines Monomeren, welches durch Initiierung mit freien Radikalen polymerisierbar ist, das umfaßt
die Zugabe zu diesem Monomeren (a) einerwirksamen Menge einer Kombination von Verbindungen der Komponente (b) gemäß Anspruch 1.

**10.** Verfahren gemäß Anspruch 9 zur Verhinderung einer Verschmutzung von Verfahrensvorrichtungen einschließlich Reaktoren, Rohren, Destillationsvorrichtungen, Destillationssäulen, Cracktürmen und Wärmeübertragungsoberflächen während der Verarbeitung eines Monomeren, das durch Initiierung mit freien Radikalen polymerisierbar ist, welches die Zugabe zu dem Monomeren vor Beginn der Verarbeitung einer wirksamen Menge einer Kombination von Verbindungen der Komponente (b) gemäß Anspruch 1 umfaßt.

**11.** Verfahren gemäß Anspruch 9, das die Zugabe von 50 bis 10 000 ppm einer Mischung der Komponenten (i) und (ii) gemäß Anspruch 13 in einem Gewichtsverhältnis von 1:10 bis 1000:1 zu einem kontinuierlichen Beschickungsstrom zur Desaktivierung der autokatalytischen Polymerisation in irgendeinem Teil einer kontinuierlichen Verfahrensvorrichtung irgendeines ethylenisch ungesättigten Monomeren, das in dem Beschickungsstrom vorhanden ist, und die weitere Zugabe zu diesem Beschickungsstrom zusätzlicher 10 ppm bis 500 ppm dieser Mischung als Makeupadditiv zur Aufrechterhaltung der gewünschten Konzentration dieser Mischung in dem zu behandelnden fluiden Beschickungsstrom umfaßt.

## Revendications

**1.** Composition monomère stabilisée contre la polymérisation précoce, comprenant
(a) un monomère à insaturation éthylénique ou un mélange de monomères polymérisable par amorce radicalaire libre, et
(b) une quantité efficace, suffisante pour inhiber la polymérisation précoce du constituant (a), d'une combinaison

(i) d'un composé hétérocyclique pris de n'importe qu'elle formule A à D

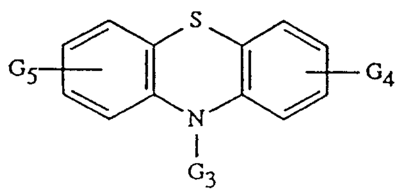

(A)

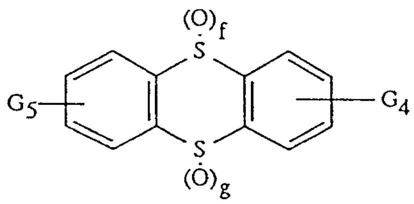

(B)

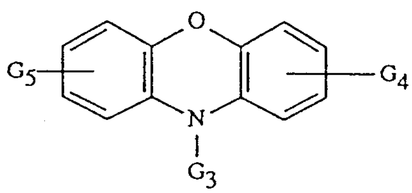

(C)

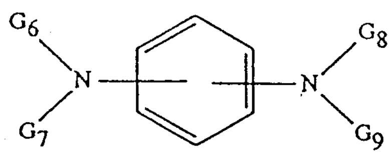

(D)

où

- $G_3$ représente l'hydrogène, un alkyle avec 1 à 4 atomes de carbone ou un alcényle avec 3 à 4 atomes de carbone,
- $G_4$ et $G_5$ indépendamment l'un de l'autre représentent l'hydrogène ou un alkyle avec 1 à 8 atomes de carbone,
- $G_6$ représente un aryle avec 6 à 10 atomes de carbone ou un aralkyle avec 7 à 15 atomes de carbone, et
- $G_7$, $G_8$ et $G_9$ indépendamment l'un de l'autre représentent l'hydrogène, un alkyle avec 1 à 18 atomes de carbone, un cycloalkyle avec 5 à 12 atomes de carbone, un aralkyle avec 7 à 15 atomes de carbone ou un alcaryle avec 7 à 15 atomes de carbone,
- f et g indépendamment l'un de l'autre valent 0, 1 ou 2, et

(ii) d'un composé ou d'un mélange de composés répondant à n'importe qu'elle formule I à XIX

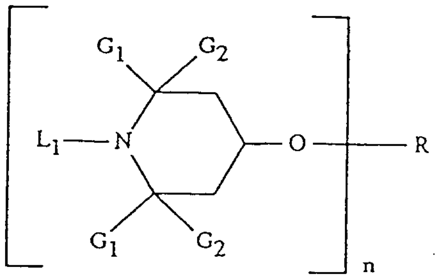

(I)

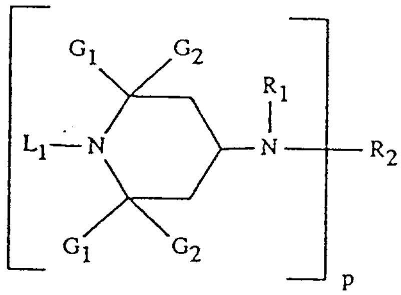

(II)

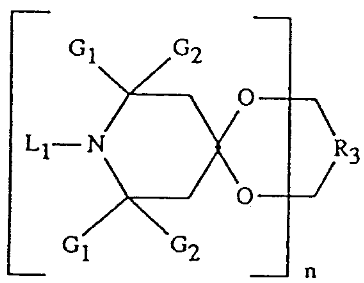

(III)

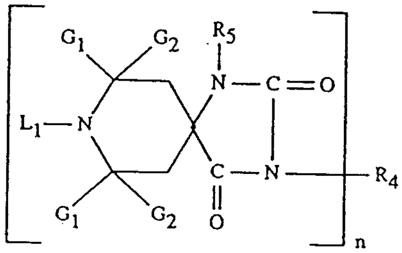

(IV)

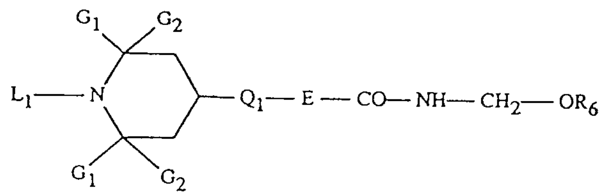

(V)

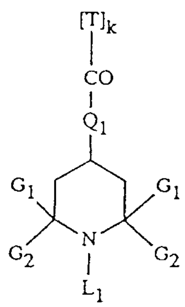

(VI)

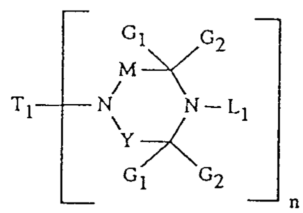

(VII)

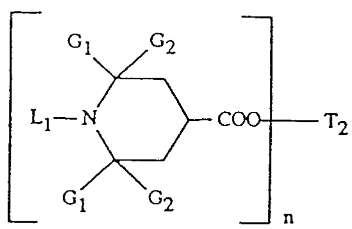

(VIII)

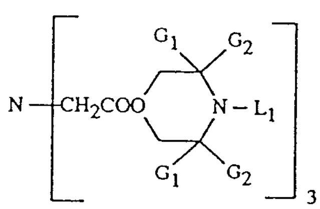

(IX)

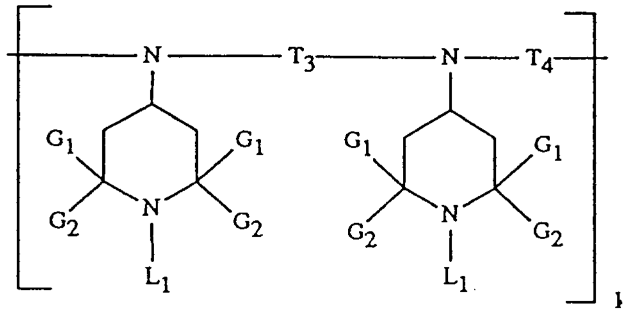

(X).

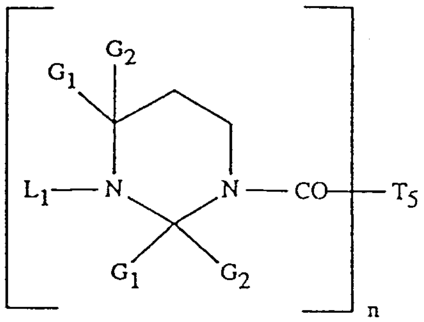

(XI)

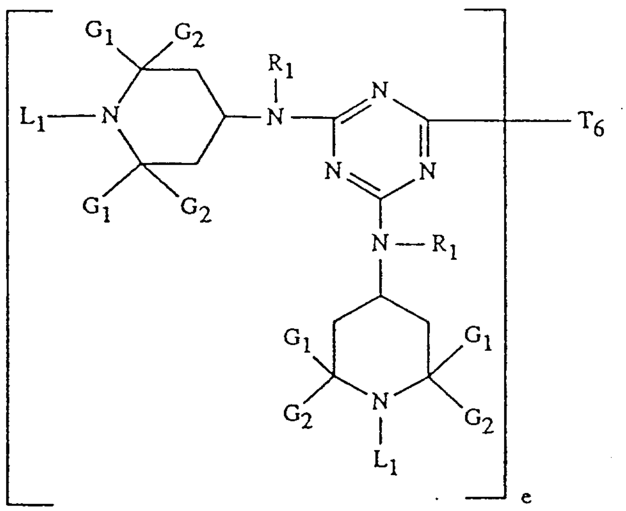

(XII)

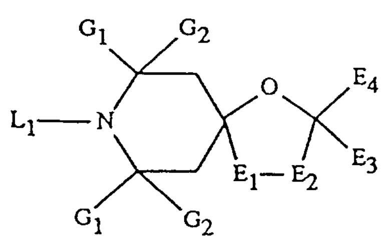

(XIII)

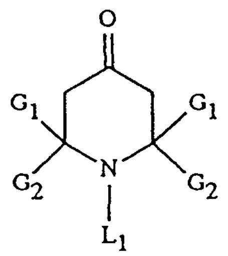

(XIV)

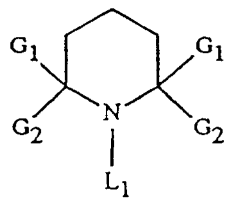

(XV)

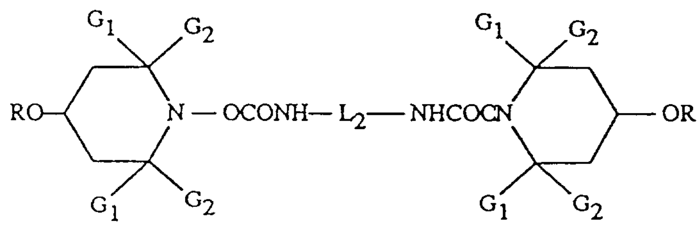

XVI

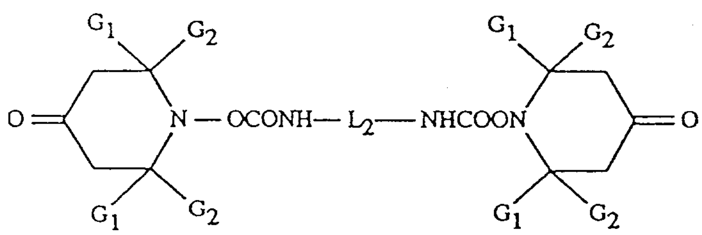

XVII

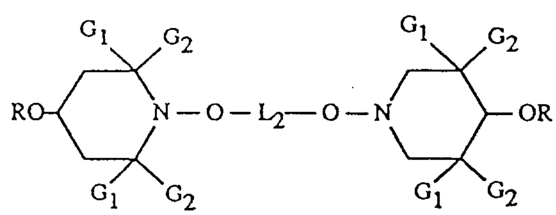

XVIII

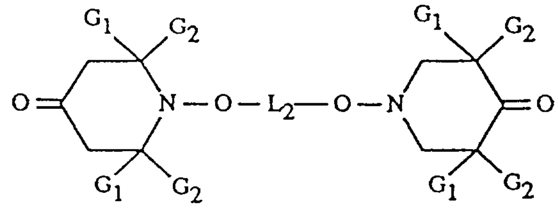

XIX

où

- $G_1$ et $G_2$, indépendamment l'un de l'autre, représentent un alkyle avec 1 à 4 atomes de carbone,
- $L_1$ représente l'hydrogène, hydroxyle, un alkyle avec 1 à 18 atomes de carbone, ledit alkyle substitué par hydroxyle, cyanoéthyle, glycidyle, aralkyle de 7 à 15 atomes de carbone ou un radical acyle monovalent d'un acide aliphatique, cycloaliphatique, araliphatique ou aromatique, $-OCONHL_3$ ou $-OL_4$, où
- $L_3$ représente l'hydrogène, un alkyle avec 2 à 18 atomes de carbone, un allyle, un

cyclohexyle, un aryle avec 6 à 10 atomes de carbone, ledit aryle substitué par 1 ou 2 groupes alkyles avec 1 à 4 atomes de carbone ou représente benzyle,

$L_4$ représente un alkyle avec 1 à 18 atomes de carbone, un cycloalkyle avec 5 à 12 atomes de carbone, un alcényle avec 2 à 18 atomes de carbone, un cycloalcényle avec 5 à 12 atomes de carbone, un aralkyle avec 7 à 15 atomes de carbone, un radical d'un hydrocarbure saturé ou insaturé bicyclique ou tricyclique avec 7 à 12 atomes de carbone ou un aryle avec 6 à 10 atomes de carbone ou un aryle avec 6 à 10 atomes de carbone ou ledit aryle substitué par un alkyle avec 1 à 4 atomes de carbone, ou $L_4$ représente $-CH_2-CH_2COOL_5$ où $L_5$ est un alkyle avec 1 à 18 atomes de carbone,

n vaut 1 ou 2,

lorsque n est 1,

R représente l'hydrogène, un alkyle en $C_1$-$C_{18}$ interrompu facultativement par 1 ou plusieurs atomes d'oxygène, cyanoéthyle, benzyle, glycidyle, un radical acyle monovalent d'un acide aliphatique, cycloaliphatique, araliphatique ou aromatique, ou un acide carbamique ou un acide contenant du phosphore ou un radical silyle monovalent ; ou

lorsque n est 2,

R représente un alkylène en $C_1$-$C_{12}$, un alcénylène en $C_4$-$C_{12}$, un xylylène, un radical acyle divalent d'un acide dicarboxylique aliphatique, cycloaliphatique, araliphatique ou aromatique, ou d'un acide dicarbamique ou d'un acide contenant du phosphore, ou un radical silyle bivalent ;

p vaut 1, 2 ou 3,

$R_1$ représente un alkyle en $C_1$-$C_{12}$, un cycloalkyle en $C_5$-$C_7$, un aralkyle en $C_7$-$C_8$, un alcanoyle en $C_2$-$C_{18}$, alcénoyle en $C_3$-$C_5$ ou benzoyle,

lorsque p vaut 1,

$R_2$ représente un alkyle en $C_1$-$C_{18}$, un cycloalkyle en $C_5$-$C_7$, un alcényle en $C_2$-$C_8$ non substitué ou substitué par un groupe cyano, carbonyle ou carbamide, ou représente le glycidyle, un groupe de formule $-CH_2CH(OH)$-Z ou de formule -CONH-Z où Z est l'hydrogène, un méthyle ou un phényle, ou

lorsque p vaut 2,

$R_2$ représente un alkylène en $C_2$-$C_{12}$, un arylène en $C_6$-$C_{12}$,xylylène, un groupe $-CH_2CH(OH)-CH_2-O-X-O-CH_2CH(OH)CH_2-$dans laquelle X est un alkylène en $C_2$-$C_{10}$, un arylène en $C_6$-$C_{15}$ ou un cycloalkylène en $C_6$-$C_{12}$ ; ou à la condition que $R_1$ ne soit pas alcanoyle, alcénoyle ou benzoyle, $R_2$ peut être aussi un radical acyle divalent d'un acide dicarbamique ou dicarboxylique aliphatique, cycloaliphatique ou aromatique, ou peut être le groupe - CO ; ou $R_1$ et $R_2$ ensemble lorsque p vaut 1, peuvent être le radical acyle cyclique d'un acide 1,2- ou 1,3-dicarboxylique aliphatique ou aromatique, ou

$R_2$ représente

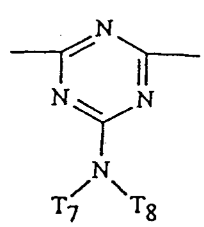

où

$T_7$ et $T_8$ indépendamment l'un de l'autre représentent l'hydrogène, un alkyle avec 1 à 18 atomes de carbone, ou $T_7$ et $T_8$ ensemble sont un alkylène avec 4 à 6 atomes de carbone ou le 3-oxapentaméthylène,

lorsque p est 3,

$R_2$ représente le 2,4,6-triazinyle,

lorsque n vaut 1,

$R_3$ représente un alkylène en $C_2$-$C_8$ ou hydroxyalkylène ou acyloxyalkylène en $C_4$-$C_{22}$ ; ou

lorsque n vaut 2,

$R_3$ représente $(-CH_2)_2C(CH_2-)_2$,

lorsque n vaut 1,

$R_4$ représente l'hydrogène, un alkyle en $C_1$-$C_{12}$, alcényle en $C_3$-$C_5$, un aralkyle en $C_7$-$C_9$, un cycloalkyle en $C_5$-$C_7$, hydroxy-alkyle en $C_2$-$C_4$, alcoxyalkyle en $C_2$-$C_6$, aryle en $C_6$-$C_{10}$, glycidyle, un groupe de formule $-(CH_2)_m-COO-Q$ ou de formule $-(CH_2)_m-O-CO-Q$ dans laquelle m vaut 1 ou 2 et Q représente un alkyle en $C_1$-$C_4$ ou phényle, ou

lorsque n vaut 2,

$R_4$ représente un alkylène en $C_2$-$C_{12}$, un arylène en $C_6$-$C_{12}$,un groupe $-CH_2CH(OH)CH_2-O-X-O-CH_2CH(OH)CH_2-$ dans lequel X représente un alkylène en $C_2$-$C_{10}$, un arylène en $C_6$-$C_{15}$ ou un cycloalkylène en $C_6$-$C_{12}$, ou un groupe $-CH_2CH(OZ_1)CH_2-(OCH_2CH(OZ_1)CH_2)_2-$ dans lequel $Z_1$ représente l'hydrogène, un alkyle en $C_1$-$C_{18}$, allyle, benzyle, alcanoyle en $C_2$-$C_{12}$ ou benzoyle,

$R_5$ représente l'hydrogène, un alkyle en $C_1$-$C_{12}$, allyle, benzyle, glycidyle ou alcoxyalkyle en $C_2$-$C_6$,

$Q_1$ représente $-N(R_7)-$ ou -O-

E représente un alkylène en $C_1$-$C_3$, le groupe $-CH_2CH(R_8)-O-$dans lequel $R_8$ est l'hydrogène, méthyle ou phényle, le groupe $-(CH_2)_3-NH-$ ou une liaison directe,

$R_7$ représente un alkyle en $C_1$-$C_{18}$, un cycloakyle en $C_5$-$C_7$,un aralkyle en $C_7$-$C_{12}$, cyanoéthyle, aryle en $C_6$-$C_{10}$, le groupe $-CH_2CH(R_8)-OH$, ou un groupe de formule

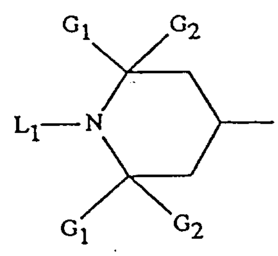

ou un groupe de formule

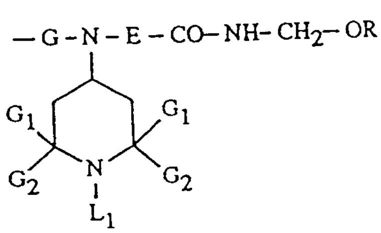

dans lequel

G représente un alkylène en $C_2$-$C_6$ ou un arylène en $C_6$-$C_{12}$, ou

$R_7$ représente un groupe $-E-CO-NH-CH_2-OR_6$.

$R_6$ représente l'hydrogène ou un alkyle en $C_1$-$C_{18}$,

La formule VI présente une unité de structure récurrente d'un polymère où T est l'éthylène ou le 1,2-propylène, ou un groupe de structure répétitif dérivé d'un copolymère d'une $\alpha$-oléfine avec un acrylate ou méthacrylate d'alkyle ;

k va de 2 à 100 ;

$T_1$ a la même signification que $R_2$ lorsque t vaut 1 ou 2 ;

M et Y indépendamment l'un de l'autre représentent le méthylène ou le carbonyle ;

$T_2$ a la même signification que $R_4$, et $T_2$ est l'octaméthylène;

$T_3$ et $T_4$ indépendamment l'un de l'autre représentent un alkylène avec 2 à 12 atomes de carbone, du $T_4$ représente

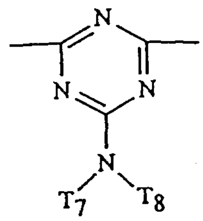

$T_6$ représente

$$-NH(CH_2)_a-N(CH_2)_b-N[(CH_2)_c-N-]_dH$$

où a, b et c indépendamment l'un de l'autre valent 2 ou 3, et d vaut 0 ou 1 ;

- e — vaut 3 ou 4 ;
- $T_5$ — a la même signification que R à la condition que $T_5$ ne puisse pas être hydrogène lorsque n est 1 ;
- $E_1$ et $E_2$, — étant différents, sont chacun oxo ou imino ;
- $E_3$ — représente l'hydrogène, un alkyle avec 1 à 30 atomes de carbone, phényle, naphtyle, ledit phényle ou ledit naphtyle substitué par du chlore ou par un alkyle avec 1 à 4 atomes de carbone ou phénylalkyle avec 7 à 12 atomes de carbone, ou ledit phénylalkyle substitué par un alkyle avec 1 à 4 atomes de carbone ; et
- $E_4$ — représente l'hydrogène, un alkyle avec 1 à 30 atomes de carbone, phényle, naphtyle ou phénylalkyle avec 7 à 12 atomes de carbone ; ou
- $E_3$ et $E_4$ — ensemble sont polyméthylène avec 4 à 17 atomes de carbone, ou ledit polyméthylène substitué par jusqu'à quatre groupes alkyles avec 1 à 4 atomes de carbone ;
- $L_2$ — est un radical divalent d'un isocyanate aliphatique, cycloaliphatique ou aromatique dont deux groupes -NCO sont éliminés, ou est un alcane-diyle avec 1 à 18 atomes de carbone ou un cyclohexane-diyle;

**2.** Composition selon la revendication 1 dans laquelle la quantité efficace est une combinaison du constituant (i) et du constituant (ii) dans un rapport pondéral de (i):(ii) de 1:10 à 1000:1, de préférence 1:1 à 10:1 et où la concentration totale en (i)+(ii) est dans le domaine de 50 à 10000 ppm, de préférence de 200 à 600 ppm, par rapport au monomère à stabiliser.

**3.** Composition selon la revendication 1, dans laquelle le constituant (a) est un monomère choisi dans le groupe comportant des hydrocarbures oléfiniques, des diènes, des monomères halogénés, des acides insaturés, des esters insaturés, des amides insaturés, des nitriles insaturés, des esters insaturés, des uréthannes acrylés et des polyesters insaturés et leurs mélanges.

**4.** Composition selon la revendication 3, dans laquelle le monomère est le styrène, le butadiène, le chlorure de vinyle, l'acide acrylique, l'acide méthacrylique, l'acétate de vinyle, l'acrylate de 2-hydroxyéthyle, le méthacrylate de 2-hydroxyéthyle, le triacrylate de triméthylolpropane, l'acrylate ou le méthacrylate de méthyle du polyéthylène-glycol, de préférence le styrène, le butadiène ou l'acide méthacrylique.

**5.** Composition selon la revendication 1 dans laquelle le constituant (i) est la phénothiazine.

**6.** Composition selon la revendication 1 dans laquelle le constituant (ii) répond à la formule I ou XV, de préférence à la formule I.

**7.** Composition selon la revendication 1 dans laquelle le constituant (ii) est choisi dans le groupe comportant

1-[2-(méthoxycarbonyl)éthoxy]-4-benzyloxy-2,2,6,6-tétraméthyl-pipéridine,
benzoate de 1-méthoxy-2,2,6,6-tétraméthylpipéridine-4-yle,
phtalate de bis[1-(2-(méthoxycarbonyl)éthoxy]-2,2,6,6-tétraméthyl-pipéridine-4-yle,

benzoate de 1-tert-butoxy-2,2,6,6-tétraméthylpipéridine-4-yle,
4-hydroxy-1-octyloxy-2,2,6,6-tétraméthylpipéridine,
[1-(2-(méthoxycarbonyl)éthoxy]-2,2,6,6-tétraméthylpipéridine,
benzoate de 1-méthylcyclohexyloxy-2,2,6,6-tétraméthylpipéridine-4-yle,
4-benzoyloxy-1-éthoxy-2,2,6,6-tétraméthylpipéridine,
1-carbamoyloxy-4-benzoyloxy-2,2,6,6-tétraméthylpipéridine,
4-hydroxy-1,2,2,6,6-pentaméthylpipéridine,
1-butylcarbamoyloxy-4-benzoyloxy-2,2,6,6-tétraméthylpipéridine,
benzoate de 1-α-méthylbenzyloxy-2,2,6,6-tétraméthylpipéridine-4-yle,
1,4-diméthoxy-2,2,6,6-tétraméthylpipéridine,
bis[1-(2-(méthoxycarbonyl)éthoxy)-2,2,6,6-tétraméthylpipéridine-4-yloxy]-p-xylylène,
benzoate de 1-hydroxy-2,2,6,6-pipéridine-4-yle,
4-hydroxy-1-méthoxy-,2,6,6-tétraméthylpipéridine,
1-méthoxy-2,2,6,6-tétraméthylpipéridine-4-one,
1-hydroxy-2,2,6,6-tétraméthylpipéridine-4-one,
4-hydroxyéthoxy-2,2,6,6-tétraméthylpipéridine,
sébacate de bis-(1-hydroxy-2,2,6,6-tétraméthylpipéridine-4-yle) et
sébacate de bis-(octyloxy-2,2,6,6-tétraméthylpipéridine-4-yle).

**8.** Composition selon la revendication 1 qui contient de plus un autre stabilisant choisi dans le groupe comportant l'hydroquinone et l'éther monométhylique de l'hydroquinone.

**9.** Procédé pour empêcher la polymérisation précoce d'un monomère polymérisable par amorce radicalaire libre qui comprend l'addition audit monomère (a) d'une quantité efficace d'une combinaison de composés du constituant (b) selon la revendication 1.

**10.** Procédé selon la revendication 9 pour empêcher le colmatage de l'équipement de procédé y compris les réacteurs, les tuyauteries, les appareils de distillation, les colonnes de distillation, les tours de craquage et les surfaces échangeuses de chaleur pendant le traitement d'un monomère polymérisable par amorce à radicaux libres qui comprend

l'addition audit monomère, avant le début du traitement, d'une quantité efficace d'une combinaison des composés du constituant (b) selon la revendication 1.

**11.** Procédé selon la revendication 9 qui comprend

l'addition de 50 à 10 000 ppm d'un mélange de constituants (i) et (ii), selon la revendication 13 dans un rapport pondéral de 1:10 à 1000:1 à un flux d'alimentation continu pour désactiver la polymérisation autocalytique dans n'importe quelle partie de l'équipement de procédé continu, de n'importe quel monomère à insaturation éthylénique présent dans le courant alimentaire, et

de plus l'addition audit courant alimentaire de 10 ppm à 500 ppm supplémentaires dudit mélange en tant qu'additif complémentaire pour maintenir la concentration désirée endit mélange dont le courant alimentaire fluide à traiter.